# EUROPEAN PATENT SPECIFICATION

(11) **EP 4 046 491 B1**
(45) Date of publication and mention of the grant of the patent: **29.04.2026**
(21) Application number: 22166673.8
(22) Date of filing: 28.02.2017
(51) Int. Cl.: A01N 37/44, A01N 37/06, A01P 17/00

(54) **CONSUMER CARE PRODUCT**
VERBRAUCHERPFLEGEPRODUKT
PRODUIT DE SOIN POUR LE CONSOMMATEUR

(30) Priority: 29.02.2016 US 201662301364 P
(43) Date of publication of application: 24.08.2022
(62) Divisional of application: 17760571.4
(73) Proprietor: FMC Corporation, Philadelphia, PA 19104 (US)
(72) Inventor: BLACK, Bruce C, Yardley 19067 (US); ALBRIGHT, Robert B, Lansdale 19446 (US); CALDWELL, Nathan D, Morrisville 19067 (US); RICHMAN, Dina L, Philadelphia 19134 (US)
(74) Representative: Murgitroyd & Company

(56) References cited:
- WO-A1-2015/009905
- WO-A1-2015/009911
- US-A- 5 296 226
- UNITED STATES ENVIRONMENTAL PROTECTION AGENCY: "Bird Shield Repellent Corporation", 7 November 2013 (2013-11-07), XP055926542, Retrieved from the Internet <URL:https://www3.epa.gov/pesticides/chem_search/ppls/066550-00001-20131107.pdf> [retrieved on 20220531]

## Description

### FIELD OF THE INVENTION

This invention relates to a consumer care product having a pest repellent composition comprising a fatty acid and an anthranilate ester.

### BACKGROUND OF THE INVENTION

Insect and acarid pests can cause severe damage to crops and horticultural plants. Moreover insects and pests can carry agents that cause disease in both people and animals. For example, mosquitoes can carry viruses that cause encephalitis, and ticks can carry bacteria that cause Lyme disease. One method of controlling such pests involves the application of chemicals which repels such pests from a given environment. Thus, chemicals such as N,N-diethyl-m-toluamide (also known as DEET) is employed in repellents to protect individuals from mosquitoes, ticks and other similar pests. Other chemicals which affect insect behavior are used to attract or repel insects from a given environment in order to enhance the effectiveness of insecticides, either by attracting insects to the area where insecticides can be more effectively employed, or by repelling them from areas where insecticides are inefficient.

Among the compounds which have been employed to influence insect behavior so as to cause them to move to a different environment is methyl anthranilate. Thus, US Patent 6,958,146 (Askham et al), US Patent 7,867,479 (Dunham et al) and US Patent 8,092,790 (Dunham et al) all disclose the use of methyl anthranilate in the form of the commercial product BIRDSHIELD^{™} to induce insects to migrate from one environment to another (see, for example, Column 9, lines 26-33 of US 7,867,479). WO2015/009911A1 and WO2015/009905A1 are also useful in understanding the present invention.

Anthranilate esters such as methyl anthranilate have long been known to be useful as bird repellents. Thus, US Patent 2,967,128 (Kare) describes the use of such compounds to deter both domestic and wild birds from eating seeds, berries, grains, fruits and the like. Further, such compounds have found to be insect attractants -- for example, US Patent 5,296,226 (Askham) states (at Column 3, lines 20-23) that "insects are readily attracted to dimethyl and methyl anthanilate. Crops relatively free of insects were quickly reinfested after being treated with either material." This finding is supported by the disclosures in US Patent 6,958,146 (Askham et al), US Patent 7,867,479 (Dunham et al) and US Patent 8,092,790 (Dunham et al) which show in Table 1 of such publications that sticky traps containing methyl anthranilate quickly became covered with hundreds of insects. The sole exception presented in these patents are house flies (*Musca domesticae*) which were repelled by the use of methyl anthranilate.

Although US Patents 6,958,146, 7,867,479 and 8,092,790 all describe BIRDSHIELD^{™} as being a mixture of methyl anthranilate with a fatty acid, it is noted that the label for such product indicates that it is covered by the claims of US Patent 5,296,226. This patent is directed to bird repellant compositions comprising an anionic surfactant consisting of an alkyl metal salt of a fatty acid; rather than a fatty acid in acid form. According to this publication, the addition of such fatty acid salts results in the formation of micelles of such anthranilate compounds, permitting a more even distribution of such compounds on the surface treated and enhancing their efficacy as bird repellents.

Given that compositions comprising an anthranilate ester and a fatty acid salt will attract most insects and will repel birds, it is completely unexpected that a composition comprising an anthranilate ester and a fatty acid in acid form will effectively repel many insect species as well as acarids, without repelling birds, making them useful for a number of repellent purposes including protecting bird feeders from unwanted insects.

Furthermore, many substances are applied topically to the skin or mucous membranes of humans or animals (hereafter "skin") in order to alter the subject's appearance, to protect the subject from the environment, or to produce a biological change in the skin or other tissue for therapeutic, preventive or cosmetic purposes. These substances may generically be termed "consumer care products" and include such topically applied substances as cosmetics, over-the-counter and prescription topical drugs, and a variety of other products such as soaps and detergents.

Consumer care products occur in a variety of forms, including solids, liquids, suspensions, semisolids (such as creams, gels, pastes or "sticks"), powders or finely dispersed liquids such as sprays or mists. Examples of consumer care products commonly classified as "cosmetics" include skin care products such as creams, lotions, moisturizers and "treatment cosmetics" such as exfoliants and/or skin cell renewal agents; fragrances such as perfumes and colognes, and deodorants; shaving-related products such as creams, "bracers" and aftershaves; depilatories and other hair removal products; skin cleansers, toners and astringents; pre-moistened wipes and washcloths; tanning lotions; bath products such as oils; eye care products such as eye lotions and makeup removers; foot care products such as powders and sprays; skin colorant and make-up products such as foundations, blushes, rouges, eye shadows and liners, lip colors and mascaras; lip balms and sticks; hair care and treatment products such as shampoos, conditioners, colorants, dyes, bleaches, straighteners and permanent wave products; baby products such as baby lotions, oils, shampoos, powders and wet wipes; feminine hygiene products such as deodorants and douches; skin or facial peels applied by dermatologists or cosmeticians; and others.

Examples of consumer care products commonly classified as "topical drugs" are many and varied, and include over-the-counter and/or prescription products such as antiperspirants, insect repellents, sunscreens and sunburn treatments, anti-acne agents, antibiotics, topical respiratory agents, ocular drugs such as eyedrops and saline solutions, therapeutic retinoids, anti-dandruff agents, external analgesics such as capsaicin products, topical contraceptives, topical drug delivery systems, gastrointestinal agents such as suppositories, enemas and hemorrhoid treatments, reproductive system agents such as vaginal treatments, oral treatments such as lozenges, and many other products with therapeutic or other effects. Other consumer care products include hand, facial and body soaps and detergents and other forms of skin cleansers, as well as household detergents and many other household products such as solvents, propellants, polishes, lubricants, adhesives, waxes and others which are either applied topically or are topically exposed to the body during normal use.

Applicants have recognized the need to develop consumer care products containing an anthranilate ester and a fatty acid salt that can effectively repel many insect species as well as acarids, without repelling birds, making them useful for a number of repellent purposes including protecting bird feeders from unwanted insects.

### SUMMARY OF THE INVENTION

In one aspect, the present invention is directed to a consumer care product as defined in claim 1. The consumer care products are for repelling insects and/or acarid pests.

The composition comprises an insect or acarid repellant described herein, where the fatty acid is selected from the group consisting of oleic acid, ricinoleic acid, linoleic acid palmitic acid, stearic acid, and any mixtures thereof.

Optionally, the fatty acid is oleic acid.

The first active ingredient is an anthranilate ester selected from the group consisting of dimethyl anthranilate, methyl anthranilate, ethyl anthranilate, phenylethyl anthranilate and menthyl anthranilate. In certain embodiments, the anthranilate ester is methyl anthranilate.

The composition further comprises an additional compound selected from the group consisting of a surfactant, emulsifying agent, a pH modifier, a thickening agent, an anti-oxidant, a preservative, a skin conditioning agent, an emollient, a humectant, a silicone moiety, an anti-inflammatory, a sun blocking agent, a topical anesthetic, vitamins, an antipruritic, an antibiotic, antifungal, an antiseptic, a vasoconstrictor, and any mixtures thereof.

In certain embodiments, the emulsifying agent is selected from the group consisting of polysaccharide ethers, polyglycosides, fatty acids, fatty alcohols, amine oxides, water-soluble cellulose, alkyl sulfonates, ethoxylated alkyl phenols, alkanolamides, betaines, zwitterionic surfactants, carboxylated alcohols, carboxylic acids, ethoxylated C12-C15 alcohols, polysorbates, behentrimonium methosulfate, , isopropyl myristate and ethoxylated castor oil or any mixtures thereof.

In certain embodiments, the thickening agent is selected from the group consisting of carboxylic acid polymers, crosslinked acrylic acid with allyl ethers of sucrose, crosslinked polyacrylate polymers, polyacrylamide polymers, polysaccharides, gums and mixtures thereof.

In certain embodiments, the polysaccharides is selected from the group consisting of cellulose, carboxymethyl hydroxyethylcellulose, cellulose acetate propionate carboxylate, hydroxyethylcellulose, hydroxyethyl ethylcellulose, hydroxypropylcellulose, hydroxypropylmethylcellulose, methyl hydroxyethylcellulose, microcrystalline cellulose, sodium cellulose sulfate, chitosan and mixtures thereof.

In certain embodiments, the gum is selected from the group consisting of calcium carrageenan, carnitine, carrageenan, dextrin, gelatin, gellan gum, guar gum, guar hydroxypropyltrimonium chloride, hectorite, hydroxypropyl guar, karaya gum, kelp, locust bean gum, natto gum, potassium carrageenan, sclerotium gum, sodium carrageenan, tragacanth gum, xanthan gum and any mixtures thereof.

The composition further comprisesa pharmaceutically suitable topical carrier, optionally comprising water, glycerin, lecithin a fatty acid or any combinations thereof.

**In** certain embodiments, the fatty acid is selected from the group consisting of oleic acid, ricinoleci acid, linoleic acid, palmitic acid, steric acid or any mixtures thereof.

**In** certain embodiments, the sun blocking agent is selected from the group consisting of PABA, PABA esters, butyl PABA, ethyl PABA, ethyl dihydroxypropyl PABA, benzophenones, avobenzone, cinnamates, salicylates anthranilates, ethyl urocanate, homosalate, dibenzoylmethanes, octocrylene, methylbenzylidene camphor, kaolin, talc, petrolatum and metal oxides or any mixtures thereof.

Another aspect of the present invention is a non-therapeutic method for repelling insects or aracrids by applying a composition as defined in claim 9.

In further embodiments of this invention, the composition is deployed by: applying the composition topically to an article of clothing of a human; or applying the composition topically to skin or hair of a human; or applying the composition topically to skin or fur of an animal; or laundering an article of clothing of a human with a detergent or fabric softener or both that comprises the composition; or drying an article of clothing of a human with a fabric softener that comprises the composition; or applying the composition to bedding, bed boards, bed slats, a mattress, box springs, furniture, carpeting, baseboards or flooring or a combination thereof; or spraying the composition on to the surface of bedding, bed boards, bed slats, a mattress, box springs, furniture or carpeting; or injecting the composition into the mattress, box springs, furniture or carpeting or a combination thereof; or deploying an absorbent substrate or gel containing the composition in the vicinity of bed boards, bed slats, a mattress, box springs, furniture or carpeting so that vapors from the composition come into contact with a surface of the bed boards, bed slats, a mattress, box springs, furniture or carpeting; or injecting the composition into a wall space.

Another aspect of this invention is the composition comprising an insect or acarid repellant described herein wherein the composition is formulated as a personal care or cosmetic composition.

In certain embodiments, the composition is formulated as a product selected from among insect repellents, skin care products, hair care products, and cleansing products.

### DETAILED DESCRIPTION OF THE INVENTION

In one aspect, the present invention is directed to a consumer care product comprising an insect- or acarid-repellant composition comprising an insect and/or acarid repellant amount of a composition as defined in claim 1. In this application, the terms "composition" and "formulation" are interchangeable.

The anthranilate esters which may be employed include those compounds described in US Patent 2,967,128, Preferred anthranilate esters are dimethyl anthranilate and methyl anthranilate, with methyl anthranilate being particularly preferred.

The compositions of this invention may further comprise additional additives conventionally employed in agricultural applications, provided that such additives in the composition are not present in a form that will cause the fatty acid to convert into a salt or other form of surfactant. In some embodiments, this may be accomplished by avoiding the addition of amines such as monoethanolamine, diethanolamine and triethanolamine.

Illustrative of further components which may be included in the compositions of this invention are antioxidant agents which serve to substantially prolong the desirable action of the fatty acid. Such antioxidant agent(s) protect the chemical and physical integrity of the fatty acid against reaction with oxygen and air pollution alone or in the presence of light. There exists an abundance of suitable antioxidants consisting of commercial and specialty chemicals and their combinations, mixtures and proprietary compositions that are well known to those educated in the art.

In certain embodiments, one particular antioxidant agent is selected from a group that includes, but is not limited to, ascorbic acid palmitate, ascorbyl palmitate, butylated p-cresol, tert-butylhydroquinone, butylated hydroquinone monomethyl ether, butylhydroxy-anisole, butylhydroxytoluene, propyl gallate and a tocopherol and combinations thereof. The amount of the antioxidant is preferably from 0.001 - 0.1 % by weight of the total composition.

While the composition can be used neat, i.e. undiluted, it is preferably employed in a diluted form. For example, the composition can be dissolved in a suitable solvent, such as a C₁-C₄ alcohol (for example, methanol, ethanol, isopropyl alcohol, butanol), a ketone such as acetone, an ester such as ethyl acetate or isopropyl myristate, a refined petroleum distillate solution (for example Sunspray^{®} 6E brand from Sunoco Inc.) or other non-reactive solvent that will evaporate, preferably in a short period of time, leaving the active mixture of fatty acid and anthranilate ester. The composition can be formulated as an Emulsifiable Concentrate (EC) with adjuvants, surfactants, stabilizers and preservatives, to be diluted with water for spray application or for application to surfaces such as mopping onto floors, wiping countertops and the like. The repellant compositions can also be formulated as aerosols for application from pressurized containers. The repellant composition can be applied by any typical method, including but not limited to spraying, painting, or dipping the object to be covered in the composition.

Another aspect of this invention is directed to a composition comprising an insect or acrid repellant provided herein, further comprising at least one additional active ingredient selected from the group consisting of pharmaceutically active agents, insecticides, pesticides, fungicides, herbicides, fertilizers, repellant and combinations thereof. Suitable additional active ingredients for the insect or acrid repellant provided herein include the following:

Insecticides: A1) the class of carbamates consisting of aldicarb, alanycarb, benfuracarb, carbaryl, carbofuran, carbosulfan, methiocarb, methomyl, oxamyl, pirimicarb, propoxur and thiodicarb; A2) the class of organophosphates consisting of acephate, azinphos-ethyl, azinphos-methyl, chlorfenvinphos, chlorpyrifos, chlorpyrifos-methyl, demeton-S-methyl, diazinon, dichlorvos/DDVP, dicrotophos, dimethoate, disulfoton, ethion, fenitrothion, fenthion, isoxathion, malathion, methamidaphos, methidathion, mevinphos, monocrotophos, oxymethoate, oxydemeton-methyl, parathion, parathion-methyl, phenthoate, phorate, phosalone, phosmet, phosphamidon, pirimiphos-methyl, quinalphos, terbufos, tetrachlorvinphos, triazophos and trichlorfon; A3) the class of cyclodiene organochlorine compounds such as endosulfan; A4) the class of fiproles consisting of ethiprole, fipronil, pyrafluprole and pyriprole; A5) the class of neonicotinoids consisting of acetamiprid, chlothianidin, dinotefuran, imidacloprid, nitenpyram, thiacloprid and thiamethoxam; A6) the class of spinosyns such as spinosad and spinetoram; A7) chloride channel activators from the class of mectins consisting of abamectin, emamectin benzoate, ivermectin, lepimectin and milbemectin; A8) juvenile hormone mimics such as hydroprene, kinoprene, methoprene, fenoxycarb and pyriproxyfen; A9) selective homopteran feeding blockers such as pymetrozine, flonicamid and pyrifluquinazon; A10) mite growth inhibitors such as clofentezine, hexythiazox and etoxazole; A11) inhibitors of mitochondrial ATP synthase such as diafenthiuron, fenbutatin oxide and propargite; uncouplers of oxidative phosphorylation such as chlorfenapyr; A12) nicotinic acetylcholine receptor channel blockers such as bensultap, cartap hydrochloride, thiocyclam and thiosultap sodium; A13) inhibitors of the chitin biosynthesis type 0 from the benzoylurea class consisting of bistrifluron, diflubenzuron, flufenoxuron, hexaflumuron, lufenuron, novaluron and teflubenzuron; A14) inhibitors of the chitin biosynthesis type 1 such as buprofezin; A15) moulting disruptors such as cyromazine; A16) ecdyson receptor agonists such as methoxyfenozide, tebufenozide, halofenozide and chromafenozide; A17) octopamin receptor agonists such as amitraz; A18) mitochondrial complex electron transport inhibitors pyridaben, tebufenpyrad, tolfenpyrad, flufenerim, cyenopyrafen, cyflumetofen, hydramethylnon, acequinocyl or fluacrypyrim; A19) voltage-dependent sodium channel blockers such as indoxacarb and metaflumizone; A20) inhibitors of the lipid synthesis such as spirodiclofen, spiromesifen and spirotetramat; A21) ryanodine receptor-modulators from the class of diamides consisting of flubendiamide, the phthalamide compounds (R)-3-Chlor-N1-{2- methyl-4-[1,2,2,2-tetrafluor-1-(trifluormethyl)ethyl]phenyl}-N2-(1-methyl-2-methylsulfonylethyl)phthalamid and (S)-3-Chlor-N1-{2-methyl-4-[1,2,2,2-tetrafluor-1-(trifluormethyl)ethyl]phenyl}-N2-(1-methyl-2-methylsulfonylethyl)phthalamid, chloranthraniliprole and cyanthraniliprole; A22) compounds of unknown or uncertain mode of action such as azadirachtin, amidoflumet, bifenazate, fluensulfone, piperonyl butoxide, pyridalyl, sulfoxaflor; or A23) sodium channel modulators from the class of pyrethroids consisting of acrinathrin, allethrin, bifenthrin, cyfluthrin, lambda-cyhalothrin, cypermethrin, alpha-cypermethrin, beta-cypermethrin, zeta-cypermethrin, deltamethrin, esfenvalerate, etofenprox, fenpropathrin, fenvalerate, flucythrinate, tau-fluvalinate, permethrin, silafluofen and tralomethrin.

Fungicides: B1) azoles selected from the group consisting of bitertanol, bromuconazole, cyproconazole, difenoconazole, diniconazole, enilconazole, epoxiconazole, fluquinconazole, fenbuconazole, flusilazole, flutriafol, hexaconazole, imibenconazole, ipconazole, metconazole, myclobutanil, penconazole, propiconazole, prothioconazole, simeconazole, triadimefon, triadimenol, tebuconazole, tetraconazole, triticonazole, prochloraz, pefurazoate, imazalil, triflumizole, cyazofamid, benomyl, carbendazim, thia- bendazole, fuberidazole, ethaboxam, etridiazole and hymexazole, azaconazole, diniconazole-M, oxpoconazol, paclobutrazol, uniconazol, 1-(4-chlorophenyl)-2-([1,2,4]triazol-1-yl)-cycloheptanol and imazalilsulfphate; B2) strobilurins selected from the group consisting of azoxystrobin, dimoxystrobin, enestroburin, fluoxastrobin, kresoxim-methyl, methominostrobin, orysastrobin, picoxystrobin, pyraclostrobin, trifloxystrobin, enestroburin, methyl (2-chloro-5-[1-(3-methylbenzyloxyimino)ethyl]benzyl)carbamate, methyl (2-chloro-5-[1-(6-methylpyridin-2-ylmethoxyimino)ethyl]benzyl)carbamate and methyl 2-(ortho-(2,5-dimethylphenyloxymethylene)- phenyl)-3-methoxyacrylate, 2-(2-(6-(3-chloro-2-methyl-phenoxy)-5-fluoro-pyrimidin-4-yloxy)-phenyl)-2-methoxyimino-N-methylacetamide and 3-methoxy-2-(2-(N-(4-methoxyphenyl)cyclopropanecarboximidoylsulfanylmethyl)-phenyl)-acrylic acid methyl ester; B3) carboxamides selected from the group consisting of carboxin, benalaxyl, benalaxyl-M, fenhexamid, flutolanil, furametpyr, mepronil, metalaxyl, mefenoxam, ofurace, oxadixyl, oxycarboxin, penthiopyrad, isopyrazam, thifluzamide, tiadinil, 3,4-dichloro-N-(2-cyanophenyl)isothiazole-5-carboxamide, dimethomorph, flumorph, flumetover, fluopicolide (picobenzamid), zoxamide, carpropamid, diclocymet, mandipropamid, N-(2- (4-[3-(4-chlorophenyl)prop-2-ynyloxy]-3-methoxyphenyl)ethyl)-2- methanesulfonyl-amino-3-methylbutyramide, N-(2-(4-[3-(4-chlorophenyl)prop-2-ynyloxy]-3-methoxy-phenyl)ethyl)-2-ethanesulfonylamino-3-methylbutyramide, methyl 3-(4-chlorophenyl)-3-(2-isopropoxycarbonyl-amino-3-methyl-butyrylamino)propionate, N-(4'-bromobiphenyl-2-yl)-4-difluoromethyl-methylthiazole-.-carboxamide, N-(4'-trifluoromethyl- biphenyl-2-yl)-4-difluoromethyl-2-methylthiazole-5-carboxamide, N-(4'-chloro-3'-fluorobiphenyl-2-yl)-4-difluoromethyl-2-methyl-thiazole-5-carboxamide, N-(3,4'-dichloro-4-fluorobiphenyl-2-yl)-3-difluoro-methyl-1-methyl-pyrazole-4-carboxamide, N-(3',4'-dichloro-5-fluorobiphenyl-2-yl)-3-difluoromethyl-1-methylpyrazole-4-carboxamide, N-(2-cyano-phenyl)- 3,4-dichloroisothiazole-5-carboxamide, 2-amino-4-methylthiazole-5-carboxanilide, 2-chloro-N-(1,1,3-trimethyl-indan-4-yl)-nicotinamide, N-(2-(1,3-dimethylbutyl)-phenyl)-1,3-dimethyl-5-fluoro-1H-pyrazole-4-carboxamide, N-(4'-chloro-3',5-difluoro-biphenyl-2-yl)-3-difluoromethyl-1-methyl-I H-pyrazole-4-carboxamide, N-(4'-chloro-3',5-difluoro-biphenyl- 2-yl)-3-trifluoromethyl-1 -methyl-1H-pyrazole-4-carboxamide, N-(3',4'- dichloro-5-fluoro-biphenyl-2-yl)-3-trifluoromethyl-1-methyl-1H-pyrazole-4-carboxamide, N-(3',5-difluoro-4'-methylbiphenyl-2-yl)-3-difluoromethyl-1-methyl-1H-pyrazole-4-carboxamide, N-(3',5-difluoro-4'-methyl-biphenyl-2-yl)-3-trifluoromethyl-1-methyl-1H-pyrazole-4-carboxamide, N-(cis-2-bicyclopropyl-2-yl-phenyl)-3-difluoromethyl-1-methyl-1H-pyrazole-4-carboxamide, N-(trans-2-bicyclopropyl-2-yl-phenyl)-3-difluoro-methyl-1-methyl-1H-pyrazole-4-carboxamide, fluopyram, N-(3-ethyl-3,5-5- trimethylcyclohexyl)-3-formylamino-2-hydroxy-benzamide, oxytetracyclin, silthiofam, N-(6-methoxy-pyridin-3-yl)cyclopropanecarboxamide, 2-iodo-N-phenyl-benzamide, N-(2-bicyclo-propyl-2-yl-phenyl)-3-difluormethyl-1-methylpyrazol-4-ylcarboxamide, N-(3',4',5'-trifluorobiphenyl-2-yl)-1,3-dimethylpyrazol-4-ylcarboxamide, N-(3',4',5'-trifluorobiphenyl-2-yl)-1,3-dimethyl-5-fluoropyrazol-4-ylcarboxamide, N-(3',4',5'-trifluorobiphenyl-2-yl)-5-chloro-1,3-dimethyl-pyrazol-4-ylcarboxamide, N-(3',4',5'-trifluorobiphenyl-2-yl)-3-fluoromethyl-1-methylpyrazol-4-ylcarboxamide, N-(3',4',5'- trifluorobiphenyl-2-yl)-3-(chlorofluoromethyl)-1-methylpyrazol-4-ylcarboxamide,N-(3',4',5'-trifluorobiphenyl-2-yl)-3-difluoromethyl-1-methylpyrazol-4-ylcarboxamide, N-(3',4',5'-trifluorobiphenyl-2-yl)-3-difluoromethyl-5-fluoro-1-methylpyrazol-4-ylcarboxamide, N-(3',4',5'-trifluorobiphenyl-2-yl)-5-chloro-3-difluoromethyl-1-methylpyrazol-4-ylcarboxamide, N- (3', 4', 5'-trifluorobiphenyl-2-yl)-3-(chlorodifluoromethyl)-1-methylpyrazol-4-ylcarboxamide, N-(3',4',5'-trifluorobiphenyl-2-yl)-1-methyl-3-trifluoromethylpyrazol-4-ylcarboxamide, N-(3',4',5'-trifluorobiphenyl-2-yl)- 5-fluoro-1-methyl-3-trifluoromethylpyrazol-4-ylcarboxamide, N-(3',4',5'-trifluorobiphenyl-2-yl)-5-chloro-1-methyl-3-trifluoromethylpyrazol-4-ylcarboxamide, N-(2',4',5'-trifluorobiphenyl-2-yl)-1,3-dimethylpyrazol-4-ylcarboxamide, N-(2',4',5'-trifluorobiphenyl-2-yl)-1,3-dimethyl-5-fluoropyrazol-4-ylcarboxamide, N-(2',4',5'- trifluorobiphenyl-2-yl)-5-chloro-1 ,3-dimethylpyrazol-4-ylcarboxamide, N- (2',4',5'-trifluorobiphenyl-2-yl)-3-fluoromethyl-1-methylpyrazol-4-ylcarboxamide, N-(2',4',5'-trifluorobiphenyl-2-yl)-3-(chlorofluoromethyl)-1-methylpyrazol-4-ylcarboxamide,N-(2',4',5'-trifluorobiphenyl-2-yl)-3-difluoromethyl-1-methylpyrazol-4-ylcarboxamide, N-(2',4',5'-trifluorobiphenyl-2-yl)-3-difluoromethyl-5- fluoro-1-methylpyrazol-4-ylcarboxamide, N-(2',4',5'-trifluorobiphenyl-2- yl)-5-chloro-3-difluoromethyl-1-methylpyrazol-4-ylcarboxamide, N-(2',4',5'-trifluorobiphenyl-2-yl)-3-(chlorodifluoromethyl)-1-methylpyrazol-4-ylcarboxamide, N-(2',4',5'-trifluorobiphenyl-2-yl)-1-methyl-3-trifluoromethylpyrazol-4-ylcarboxamide, N-(2',4',5'-trifluorobiphenyl-2-yl)-5-fluoro-1-methyl-3-trifluoromethylpyrazol-4-ylcarboxamide, N-(2',4',5'-trifluorobiphenyl-2-yl)-5-chloro-1-methyl-3-trifluoromethylpyrazol-4-ylcarboxamide, N-(3',4'-dichloro-3-fluorobiphenyl-2-yl)-1-methyl-3-trifluoromethyl-1H-pyrazole-4-carboxamide, N-(3',4'-dichloro-3-fluorobiphenyl-2-yl)-1-methyl-3-difluoromethyl-1H-pyrazole-4-carboxamide, N-(3',4'-difluoro-3-fluorobiphenyl-2-yl)-1-methyl-3-trifluoromethyl-1H-pyrazole-4-carboxamide, N-(3',4'-difluoro-3-fluorobiphenyl-2-yl)-1-methyl-S-difluoromethyl-1H-pyrazole-4-carboxamide, N-(3'-chloro-4'-fluoro-3-fluorobiphenyl-2-yl)-1-methyl-3-difluoromethyl-1H-pyrazole-4-carboxamide, N-(3',4'-dichloro-4-fluorobiphenyl-2-yl)-1-methyl-3-trifluoromethyl-1H-pyrazole-4-carboxamide, N-(3',4'-difluoro-4-fluorobiphenyl-2-yl)-1-methyl-S-trifluoromethyl-1H-pyrazole-4-carboxamide, N-(3',4'-dichloro-4-fluorobiphenyl-2-yl)-1-methyl-3-difluoromethyl-1H-pyrazole-4-carboxamide, N-(3',4'-difluoro-4-fluorobiphenyl-2-yl)-1-methyl-3-difluoromethyl-1H- pyrazole-4-carboxamide, N-(3'-chloro-4'-fluoro-4-fluorobiphenyl-2-yl)-1-methyl-S-difluoromethyl-1H-pyrazole-4-carboxamide, N-(3',4'-dichloro-5- fluorobiphenyl-2-yl)-1-methyl-3-trifluoromethyl-1H-pyrazole-4-carboxamide, N-(3',4'-difluoro-5-fluorobiphenyl-2-yl)-1-methyl-3-trifluoromethyl-1H-pyrazole-4-carboxamide, N-(3',4'-dichloro-5-fluorobiphenyl-2-yl)-1-methyl-S-difluoromethyl-1H-pyrazole-4-carboxamide, N-(3',4'-difluoro-5-fluorobiphenyl-2-yl)-1-methyl-3-difluoromethyl-1H-pyrazole-4-carboxamide, N-(3',4'-dichloro-5-fluorobiphenyl-2-yl)-1,3-dimethyl-1H-pyrazole-4-carboxamide, N-(3'-chloro-4'-fluoro-5-fluorobiphenyl-2-yl)-1-methyl-3- difluoromethyl-1H-pyrazole-4-carboxamide, N-(4'-fluoro-4-fluorobiphenyl-2-yl)-1 -methyl-3-trifluoromethyl-1H-pyrazole-4-carboxamide, N-(4'-fluoro- 5-fluorobiphenyl-2-yl)-1-methyl-3-trifluoromethyl-1H-pyrazole-4-carboxamide,N-(4'-chloro-5-fluorobiphenyl-2-yl)-1-methyl-3-trifluoromethyl-1H- pyrazole-4-carboxamide, N-(4'-methyl-5-fluorobiphenyl-2-yl)-1-methyl-3-trifluoromethyl-1H-pyrazole-4-carboxamide, N-(4'-fluoro-5- fluorobiphenyl-2-yl)-1,3-dimethyl-1H-pyrazole-4-carboxamide, N-(4'-chloro-5-fluorobiphenyl-2-yl)-1,3-dimethyl-1H-pyrazole-4-carboxamide, N-(4'-methyl-5-fluorobiphenyl-2-yl)-1,3-dimethyl-1H-pyrazole-4-carboxamide, N-(4'-fluoro-6-fluorobiphenyl-2-yl)-1-methyl-3-trifluoromethyl-1H- pyrazole-4-carboxamide, N-(4'-chloro-6-fluorobiphenyl-2-yl)-1-methyl-3- trifluoromethyl-1H-pyrazole-4-carboxamide, N-[2-(1,1,2,3,3,3- hexafluoropropoxy)-phenyl]-3-difluoromethyl-1-methyl-1H-pyrazole-4-carboxamide, N-[4'-(trifluoromethylthio)-biphenyl-2-yl]-3-difluoromethyl-1-methyl-1H-pyrazole-4-carboxamide and N-[4'-(trifluoromethylthio)-biphenyl-2-yl]-1-methyl-3-trifluoromethyl-1-methyl-1H-pyrazole-4-carboxamide; B4) heterocyclic compounds selected from the group consisting of fluazinam, pyrifenox, bupirimate, cyprodinil, fenarimol, ferimzone, mepanipyrim, nuarimol, pyrimethanil, triforine, fenpiclonil, fludioxonil, aldimorph, dodemorph, fenpropimorph, tridemorph, fenpropidin, iprodione, procymidone, vinclozolin, famoxadone, fenamidone, octhilinone, proben- azole, 5-chloro-7-(4-methyl-piperidin-1-yl)-6-(2,4,6-trifluorophenyl)-[1,2,4]triazolo[1,5-a]pyrimidine, anilazine, diclomezine, pyroquilon, proquinazid, tricyclazole, 2-butoxy-6-iodo-3-propylchromen-4-one, acibenzolar-S-methyl, captafol, captan, dazomet, folpet, fenoxanil, quinoxyfen, N,N-dimethyl-3-(3-bromo-6-fluoro-2-methylindole-1-sulfonyl)- [1,2,4]triazole-1-sulfonamide, 5-ethyl-6-octyl-[1,2,4]triazolo[1,5-a]pyrimidin-2,7-diamine, 2,3,5,6-tetrachloro-4-methanesulfonyl-pyridine, 3,4,5-trichloro-pyridine-2,6-di-carbonitrile, N-(1-(5-bromo-3-chloro-pyridin-2-yl)-ethyl)-2,4-dichloro-nicotinamide, N-((5-bromo-3-chloro pyridin-2-yl)-methyl)-2,4-dichloro-nicotinamide, diflumetorim, nitrapyrin, dodemorphacetate, fluoroimid, blasticidin-S, chinomethionat, debacarb, difenzoquat, difenzoquat-methylsulphate, oxolinic acid and piperalin; B5) carbamates selected from the group consisting of mancozeb, maneb, metam, methasulphocarb, metiram, ferbam, propineb, thiram, zineb, ziram, diethofencarb, iprovalicarb, benthiavalicarb, propamocarb, propamocarb hydrochlorid, 4-fluorophenyl N-(1-(1-(4-cyanophenyl)-ethanesulfonyl)but-2-yl)carbamate, methyl 3-(4-chloro-phenyl)-3-(2-isopropoxycarbonylamino-3-methyl-butyrylamino)propanoate; or B6) other fungicides selected from the group consisting of guanidine, dodine, dodine free base, iminoctadine, guazatine, antibiotics: kasugamycin, streptomycin, polyoxin, validamycin A, nitrophenyl derivatives: binapacryl, dinocap, dinobuton, sulfur-containing heterocyclyl compounds: dithianon, isoprothiolane, organometallic compounds: fentin salts, organophosphorus compounds: edifenphos, iprobenfos, fosetyl, fosetyl-aluminum, phosphorous acid and its salts, pyrazophos, tolclofos-methyl, organochlorine compounds: dichlofluanid, flusulfamide, hexachlorobenzene, phthalide, pencycuron, quintozene, thiophanate-methyl, tolylfluanid, others: cyflufenamid, cymoxanil, dimethirimol, ethirimol, furalaxyl, metrafenone and spiroxamine, guazatine-acetate, iminoctadine-triacetate, iminoctadine-tris(albesilate), kasugamycin hydrochloride hydrate, dichlorophen, pentachlorophenol and its salts, N-(4- chloro-2-nitro-phenyl)-N-ethyl-4-methyl-benzenesulfonamide, dicloran, nitrothal-isopropyl, tecnazen, biphenyl, bronopol, diphenylamine,mildiomycin, oxincopper, prohexadione calcium, N- (cyclopropylmethoxyimino-(6-difluoromethoxy-2,3-difluoro-phenyl)- methyl)-2-phenyl acetamide, N'-(4-(4-chloro-3-trifluoromethyl-phenoxy)-2,5-dimethyl-phenyl)-N-ethyl-N-methyl formamidine, N'-(4-(4-fluoro-3-trifluoromethyl-phenoxy)-2,5-dimethyl-phenyl)-N-ethyl-N-methyl formamidine, N'-(2-methyl-5-trifluormethyl-4-(3-trimethylsilanyl-propoxy)-phenyl)-N-ethyl-N-methylformamidine and N'-(5-difluormethyl-2-methyl-4-(3-trimethylsilanyl-propoxy)-phenyl)-N-ethyl-N-methyl formamidine.

Herbicides: C1) acetyl-CoA carboxylase inhibitors (ACC), for example cyclohexenone oxime ethers, such as alloxydim, clethodim, cloproxydim, cycloxydim, sethoxydim, tralkoxydim, butroxydim, clefoxydim or tepraloxydim; phenoxyphenoxypropionic esters, such as clodinafop-propargyl, cyhalofop-butyl, diclofop-methyl, fenoxaprop-ethyl, fenoxaprop-P-ethyl, fenthiapropethyl, fluazifop-butyl, fluazifop-P-butyl, haloxyfop-ethoxyethyl, haloxyfop-methyl, haloxyfop-P-methyl, isoxapyrifop, propaquizafop, quizalofop-ethyl, quizalofop-P-ethyl or quizalofop-tefuryl; or arylaminopropionic acids, such as flam prop-methyl or flamprop-isopropyl; C2) acetolactate synthase inhibitors (ALS), for example imidazolinones, such as imazapyr, imazaquin, imazamethabenz-methyl (imazame), imazamox, imazapic or imazethapyr; pyrimidyl ethers, such as pyrithiobac-acid, pyrithiobac-sodium, bispyribac-sodium, KIH-6127 or pyribenzoxym; sulfonamides, such as florasulam, flumetsulam or metosulam; or sulfonylureas, such as amidosulfuron, azimsulfuron, bensulfuron-methyl, chlorimuron-ethyl, chlorsulfuron, cinosulfuron, cyclosulfamuron, ethametsulfuron-methyl, ethoxysulfuron, flazasulfuron, halosulfuron-methyl, imazosulfuron, metsulfuron-methyl, nicosulfuron, primisulfuron-methyl, prosulfuron, pyrazosulfuron-ethyl, rimsulfuron, sulfometuron-methyl, thifensulfuron-methyl, triasulfuron, tribenuron-methyl, triflusulfuron-methyl, tritosulfuron, sulfosulfuron, foramsulfuron or iodosulfuron; C3) amides, for example allidochlor (CDAA), benzoylprop-ethyl, bromobutide, chlorthiamid. diphenamid, etobenzanid, fluthiamide, fosamin or monalide; C4) auxin herbicides, for example pyridinecarboxylic acids, such as clopyralid or picloram; or 2,4-D or benazolin; C5) auxin transport inhibitors, for example naptalame or diflufenzopyr; C6) carotenoid biosynthesis inhibitors, for example benzofenap, clomazone, diflufenican, fluorochloridone, fluridone, pyrazolynate, pyrazoxyfen, isoxaflutole, isoxachlortole, mesotrione, sulcotrione (chlormesulone), ketospiradox, flurtamone, norflurazon or amitrol; C7) enolpyruvylshikimate-3-phosphate synthase inhibitors (EPSPS), for example glyphosate or sulfosate; C8) glutamine synthetase inhibitors, for example bilanafos or glufosinate-ammonium; C9) lipid biosynthesis inhibitors, for example anilides, such as anilofos or mefenacet; chloroacetanilides, such as dimethenamid, S-dimethenamid, acetochlor, alachlor, butachlor, butenachlor, diethatyl-ethyl, dimethachlor, metazachlor, metolachlor, S-metolachlor, pretilachlor, propachlor, prynachlor, terbuchlor, thenylchlor or xylachlor; thioureas, such as butylate, cycloate, di-allate, dimepiperate, EPTC. esprocarb, molinate, pebulate, prosulfocarb, thiobencarb (benthiocarb), tri-allate or vemolate; or benfuresate or perfluidone; C10) mitosis inhibitors, for example carbamates, such as asulam, carbetamid, chlorpropham, orbencarb, propyzamid, propham or tiocarbazil; dinitroanilines, such as benefin, butralin, dinitramin, ethalfluralin, fluchloralin, oryzalin, pendimethalin, prodiamine or trifluralin; pyridines, such as dithiopyr or thiazopyr; or butamifos, chlorthal-dimethyl (DCPA) or maleic hydrazide; C11) protoporphyrinogen IX oxidase inhibitors, for example diphenyl ethers, such as acifluorfen, acifluorfen-sodium, aclonifen, bifenox, chlomitrofen (CNP), ethoxyfen, fluorodifen, fluoroglycofen-ethyl, fomesafen, furyloxyfen, lactofen, nitrofen, nitrofluorfen or oxyfluorfen; oxadiazoles, such as oxadiargyl or oxadiazon; cyclic imides, such as azafenidin, butafenacil, carfentrazone, carfentrazone-ethyl, cinidon-ethyl, flumiclorac-pentyl, flumioxazin, flumipropyn, flupropacil, fluthiacet-methyl, sulfentrazone or thidiazimin; or pyrazoles, such as ET-751, JV 485 or nipyraclofen; C12) photosynthesis inhibitors, for example propanil, pyridate or pyridafol; benzothiadiazinones, such as bentazone; dinitrophenols, for example bromofenoxim, dinoseb, dinoseb-acetate, dinoterb or DNOC; dipyridylenes, such as cyperquat-chloride, difenzoquat-methylsulfate, diquat or paraquat-dichloride; ureas, such as chlorbromuron, chlorotoluron, difenoxuron, dimefuron, diuron, ethidimuron, fenuron, fluometuron, isoproturon, isouron, linuron, methabenzthiazuron, methazole, metobenzuron, metoxuron, monolinuron, neburon, siduron or tebuthiuron; phenols, such as bromoxynil or ioxynil; chloridazon; triazines, such as ametryn, atrazine, cyanazine, desmein, dimethamethryn, hexazinone, prometon, prometryn, propazine, simazine, simetryn, terbumeton, terbutryn, terbutylazine or trietazine; triazinones, such as metamitron; uracils, such as bromacil, lenacil or terbacil; or biscarbamates, such as desmedipham or phenmedipham; C13) synergists, for example oxiranes, such as tridiphane; C14) CIS cell wall synthesis inhibitors, for example isoxaben or dichlobenil; C16) various other herbicides, for example dichloropropionic acids, such as dalapon; dihydrobenzofurans, such as ethofumesate; phenylacetic acids, such as chlorfenac (fenac); or aziprotryn, barban, bensulide, benzthiazuron, benzofluor, buminafos, buthidazole, buturon, cafenstrole, chlorbufam, chlorfenprop-methyl, chloroxuron, cinmethylin, cumyluron, cycluron, cyprazine, cyprazole, dibenzyluron, dipropetryn, dymron, eglinazin-ethyl, endothall, ethiozin, flucabazone, fluorbentranil, flupoxam, isocarbamid, isopropalin, karbutilate, mefluidide, monuron, napropamide, napropanilide, nitralin, oxaciclomefone, phenisopham, piperophos, procyazine, profluralin, pyributicarb, secbumeton, sulfallate (CDEC), terbucarb, triaziflam, triazofenamid or trimeturon; or their environmentally compatible salts.

The consumer care products are for repelling insects and/or acarid pests. Consumer care products may include, but are not limited to, topical compositions for application to the skin, clothing or other material to repel insect and/or acarid pests In certain embodiments of the invention, the product is a composition in liquid or semi solid form and may be aqueous, non-aqueous based or combination thereof and may be in the form of a lotion, cream, ointment, solution, foam, gel, solid stick, aerosol, bar, liquid, paste, powder, roll-on, sheet, spray, stick, tablet, suspension, emulsion, microemulsions, emusifiable concentrates, or the like. The formulation may be designed to be slowly released from a patch or be sprayed from a canister, a delivery apparatus or any suitable delivery system.

The carrier can include all carriers, topically cosmetic carriers, and pharmaceutically acceptable carriers described herein. The carrier can include water, an alcohol, an aldehyde, an alkane, an alkene, an amide, an amine, a diglyceride, an ester, an ether, a glycol ether, a fat, a fatty acid, a glycol ester, a ketone, lanolin, mineral oil, a monoglyceride, paraffin oil, a polyethylene glycol, petrolatum, a propylene carbonate, silicone, tall oils, a terpene hydrocarbon, a terpene alcohol, a triglyceride, finely divided organic solid material, finely divided inorganic solid materials and mixtures thereof. In one embodiment, the carrier can be an alcohol that is selected from among an aromatic alcohol, a C1-C6 monohydric alcohol, C2-C6 polyhydric alcohol, a polyvalent alcohol, and mixtures thereof. In one example, the carrier is an alcohol that is selected from among methanol, ethanol, propanol, butanol, sec-butanol, tert-butanol, and mixtures thereof. In a particular example, the carrier in the provided compositions is ethanol. In another particular example, the compositions contain a carrier that is isopropanol.

In a preferred embodiment, the composition comprising an insect or acarid repellant contains a topical cosmetic carrier and/or pharmaceutically acceptable carrier to form a topical cosmetic or pharmaceutical composition which can be applied to skin.

In certain embodiments, the carrier is an oil selected from the group including, but not limited to, an almond oil, avocado oil, canola oil, cashew oil, cherry seed oil, cocoa butter, coconut oil, corn oil, cottonseed oil, flaxseed oil, grape seed oil, jojoba oil, macadamia nut oil, olive oil, palm oil, palm fruit oil, peanut oil, rapeseed oil, rice bran oil, safflower oil, sesame oil, soybean oil, sunflower oil, and walnut oil and combinations thereof..

In certain embodiments, the carrier can be a diethyl ether, isopropyl ether, n-propyl ether, or a combination thereof. In one example, the carrier is acetone, a methyl ketone, a methyl benzyl ketone, a methyl ethyl ketone, a methyl isopropyl ketone, a methyl butyl ketone, an ethyl ketone, benzyl methyl ketone, and combinations thereof. In other embodiments, the carrier can be ethylene glycol, ethylene glycol monomethyl ether, ethylene glycol dimethyl ether, methylene glycol, methylene glycol monomethyl ether, methylene glycol dimethyl ether, propylene glycol, propylene glycol monomethyl ether, propylene glycol dimethyl ether, butylene glycol, butylene glycol monomethyl ether, butylene glycol dimethyl ether and combinations thereof. In another example, the carrier can be ethylene glycol, ethylene glycol monomethyl ether, ethylene glycol dimethyl ether, methylene glycol, methylene glycol monomethyl ether, methylene glycol dimethyl ether, propylene glycol, propylene glycol monomethyl ether, propylene glycol dimethyl ether, butylene glycol, butylene glycol monomethyl ether, butylene glycol dimethyl ether and combinations thereof.

In certain embodiments, the carrier can be a finely divided organic solid material or finely divided inorganic solid material. In certain preferred embodiments, the carrier can be a dust, a granule, a powder or a salt crystal. In other embodments, the carrier can be an alumina, amorphous silica, attapulgite, calcium carbonate, calcium phosphate, a clay, chalk, diatomaceous earths, fumed silica, a kaolin, kieselguhr, magnesium carbonate, microparticulate cellulose, montmorillonite, pyrophyllite, silicic acid, sodium bicarbonate, sodium carbonate, sodium phosphate, sodium pyrophosphate, talc, vermiculite, and combinations thereof.

In certain embodiments, the carrier can be an aerosol propellant that is selected from among argon, butane, carbon dioxide, a chlorofluorocarbon, dimethyl ether, a hydrocarbon, a hydrofluorocarbon, isobutane, nitrogen, propane, and a mixture thereof. In a particular example, the aerosol propellant contains difluoromethane, trifluoromethane, difluoroethane, trifluoroethane, tetrafluoroethane or octafluorocyclobutane or a combination thereof.

In other embodiments, the carrier can be a silicone oil that is selected from among cyclical silicones, linear or branched open chained silicones, and combinations thereof. In one embodiment, the silicone oil can be selected from among volatile silicones and non-volatile silicones. In another embodiment, the silicone oil is a volatile silicone oil that is selected from among cyclic polydimethylsiloxanes containing an average of from about 3 to about 9 silicon atoms and linear polydimethylsiloxanes containing an average of from about 3 to about 9 silicon atoms. In another embodiment, the silicone oil is a non-volatile silicone oil that is selected from among dimethicone copolyol, cyclomethicone, polydimethylsiloxane, cyclic dimethyl polysiloxane, aminosilicones, phenyl silicones, diphenyldimethicones, phenyltrimethicones, cyclopentasiloxane, a polymer of dimethyl-siloxane with polyoxyethylene and/or polyoxypropylene, dimethicone copolyol, cetyldimethicone copolyol, cetyl dimethicone, cetyl dimethicone copolyol and dimethiconol and combinations thereof. In further examples, the carrier is a silicone oil that is selected from among polydimethylsiloxane, phenylated silicones, decamethylcyclopentasiloxane, dodecamethylcyclohexasiloxane and octamethylcyclo-tetrasiloxane.

In certain embodiments, the carrier is selected from, but not limited to, a monoglyceride, a diglyceride, an acetylated monoglyceride, or a triglyceride or a combination thereof. In one example, the carrier contains 1-propanol, 2-propanol, 1-butanol, 2-butanol, 2-methyl-1-propanol, 1-pentanol, 2-pentanol, 3-methyl-1-butanol, 3-methyl-2-butanol, ethylene glycol, propylene glycol, 1,4-butanediol, 1,3-butanediol, 2-methyl-1,3-propanediol, 1,4-cyclohexanedimethanol, diethylene glycol, triethylene glycol, PEG-200, PEG-300, PEG-400, PEG-600, 2-methoxyethanol, 2-ethoxyethanol, 2-propoxyethanol, 2-isopropoxyethanol, 2-butoxyethanol, 1-methoxy-2-propanol, 1-ethoxy-2-propanol, 3-methoxy-1-butanol, diethylene glycol monomethyl ether, diethylene glycol monoethyl ether, diethylene glycol mono-n-propyl ether, diethylene glycol mono-isopropyl ether, diethylene glycol monobutyl ether, triethylene glycol monomethyl ether, glycerol, 3-methoxy-1,2-propanediol, or 3-ethoxy-1,2-propanediol.

In another embodiment, the carrier contains borneol, citronellol or geraniol or a combination thereof. In other embodiments, the carrier contains or is a cyclodextrin that is selected from among an α-cyclodextrin, β-cyclodextrin or γ-cyclodextrin or combinations thereof.

In certain embodiments, the consumer care product described herein includes a composition comprising an insect or acarid repellant described herein and a delivery vehicle to deliver the composition to a selected location. The delivery vehicle can contain water, an aerosol, a cream, a gel, a lotion, an oil, a spray, a soap, a detergent, a particulate or a substrate. In other embodiments, the delivery vehicle can be a substrate that is a paper, a cloth or a woven or nonwoven material. In one embodiment, the substrate is a nonwoven material that is a flexible sheet containing fibers that are adhesively or thermally bonded. The fibers can be selected from among cellulose ester, cotton, hemp, jute, linen, ramie, rayon, polyamides, polyesters polyolefins, polypropylene, polyvinyl derivatives, silk, sisal and wool and combinations thereof.

In certain embodiments, the delivery vehicle can be a gel that contains a gelling agent that is selected from among agar, a carbomer, carboxyvinyl polymers, dibenzylidene alditols, carboxypolymethylene, collagen, dextrin fatty acid esters, gelatin, hydrogenated styrene/isoprene copolymers, 12-hydroxystearic acid, K-carrageenan, gellan gum, a lower hydroxy cellulose, pectin, polyacrylic acids, styrene-ethylene/propylene block copolymers, styrene-ethylene/butylene-styrene block copolymers, sucrose fatty acid esters and a wax and combinations thereof. In other embodiments, the delivery vehicle can be a fluid that when dispensed forms a gel in situ. The fluid can contain a gelling agent that selected from among agar, an alginate, a carbomer, carboxyvinyl polymers, dibenzylidene alditols, carboxypolymethylene, collagen, dextrin fatty acid esters, gelatin, hydrogenated styrene/isoprene copolymers, 12-hydroxystearic acid, K-carrageenan, gellan gum, a lower hydroxy cellulose, pectin, polyacrylic acids, styrene-ethylene/propylene block copolymers, styrene-ethylene/butylene-styrene block copolymers, sucrose fatty acid esters and a wax and combinations thereof.

In certain embodiments, the delivery vehicle can be or contain a particulate that is selected from among an alumina, amorphous silica, attapulgite, calcium carbonate, calcium phosphate, a clay, chalk, diatomaceous earths, fumed silica, a kaolin, kieselguhr, magnesium carbonate, microparticulate cellulose, montmorillonite, pyrophyllite, silicic acid, sodium bicarbonate, sodium carbonate, sodium phosphate, sodium pyrophosphate, talc, and vermiculite, and combinations thereof.

In certain embodiments, the insect or acarid repelled by a composition provided herein can be an insect selected from among Siphonaptera insects, such as cat flea (*Ctenocephalides felis*), dog flea (*Ctenocephalides canis*), oriental rat flea (*Xenopsylla cheopis*), human flea (*Pulex irritans*), chigoe (*Tunga penetrans*) and European rat flea (*Nosopsyllus fasciatus*); Anoplura insects, such as Head louse (*Pediculus humanus capitis*), crab louse (*Pthirus pubis*), short-nosed cattle louse (*Haematopinus eurysternus*), sheep louse (*Dalmalinia ovis*), hog louse (Haematopinus suis), long-nosed cattle louse (*Linognathus vituli*), cattle biting louse (*Bovicola bovis*), poultry shaft louse (*Menopon gallinae*), poultry body louse (*Menacanthus stramineus*), little blue cattle louse (*Solenopotes capillatus*), Haematopinus spp., Linognathus spp., Pediculus spp., Phtirus spp. and Solenopotes spp.; Acarina insects, such as bush tick (*Haemaphysalis longicomis*), Haemaphysalis flava, Dermacentor taiwanicus, American dog tick (*Dermacentor variabilis*), Ixodes ovatus, Ixodes persulcatus, black legged tick (*Ixodes scapularis*), lone star tick (*Amblyomma americanum*), Boophilus microplus, Rhipicephalus sanguineus, Ixodes holocyclus, western black legged tick (*Ixodes pacificus*), Dermacentor andersoni, Ambryomma maculatum, ear mite (*Octodectes cynotis*), Psoroptes spp., Chorioptes spp., Otodectes spp., Sarcoptes spp., Notoedres spp., Knemidocoptes spp., Sacroptes scabiei, Demodex spp., follicle mite (*Demodex canis*), northern fowl mite (Omithonyssus sylviarum), poultry red mite (*Dermanyssus gallinae*), Trombicula spp., Leptotrombidium akamushi, Ornithodorus hermsi, Ornithodorus turicata, Ornithonyssus bacoti, Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Cytodites spp. and Laminosioptes spp.; Heteroptera insects, such as common bedbug (*Cimex lectularius*), tropical bedbug (*Cimex hemipterus*), Reduvius senilis, Triatoma spp. Rhodnius spp., Panstrongylus spp., and Arilus critatus; and Mallophage (*Amblycera and Ischnocera*) insects, such as Trimenopon spp., Menopon spp., Trinoton spp., Bovicola spp., Werneckiella spp., Lepikentron spp., Trichodectes spp. and Felicola spp.

In certain embodiments, the insect or acarid repelled by a composition provided herein can be an insect selected from among ants, bedbugs, carpet beetles, centipedes, chiggers, drain flies, dust mites, biting mites, earwigs, fleas, flies, gnats, hornets, lice, millipedes, mites, mosquitoes, roaches, scabies, silverfish, spiders, stinkbugs, termites, ticks, wasps, weevils and yellow jackets. The compositions comprising insect or acarid repellants provided herein, in certain embodiments, can be formulated for delivery of for at least 1 day, or at least 2 days, or at least 3 days, or at least 4 days, or at least 5 days, or at least 6 days, at least 7 days, or at least 8 days, or at least 9 days, or at least 10 days, or at least 11 days, or at least 12 days, at least 13 days, for at least 14 days, or at least 15 days, or at least 16 days, or at least 17 days, or at least 18 days, or at least 19 days, at least 20 days, or at least 21 days, or at least 22 days, or at least 23 days, or at least 24 days, or at least 25 days, at least 26 days, or at least 27 days, or at least 28 days, or at least 29 days, or at least 30 days, or at least 31 days, or at least 45 days, or at least 60 days or at least 75 days or at least 90 days.

Certain embodiments also include fabric treatment sheets containing a woven or nonwoven sheet or other substrate, including but not limited to cellulosic substrate, that is coated or impregnated with a fabric treatment composition and a composition comprising an insect or acarid repellant provided herein. The fabric treatment composition can be a detergent composition or a fabric softening composition. In some embodiments, the fabric treatment composition is a detergent composition that contains a detergent selected from among anionic surfactants, nonionic surfactants, zwitterionic surfactants, ampholytic surfactants and cationic surfactants and mixtures thereof. In other embodiments, the fabric treatment composition is a fabric softening composition that contains monomethyl trialkyl quaternaries, imidazolinium quaternaries, dimethyl alkyl benzyl quaternaries, dialkyl dimethyl quaternaries, methyl dialkoxy alkyl quaternaries, diamido amine-based quaternaries and dialkyl methyl benzyl quaternaries or (C8-C24) fatty acid amides or any combination thereof.

Certain embodiments include liquid fabric treatment compositions containing a fabric softener or fabric conditioner and a composition comprising an insect or acarid repellant provided herein. The fabric softener can be selected from among monomethyl trialkyl quaternaries, imidazolinium quaternaries, dimethyl alkyl benzyl quaternaries, dialkyl dimethyl quaternaries, methyl dialkoxy alkyl quaternaries, diamido amine-based quaternaries and dialkyl methyl benzyl quaternaries and (C8-C24) fatty acid amides and combinations thereof. In some embodiments, the fabric conditioner contains an anti-static agent, a brightening agent, a bodying agent, a soil-release agent, a wrinkle-release agent or a combination thereof. In certain embodiments, the fabric condition is an anti-static agent that contains a tertiary amine, a quaternary amine, aluminum stearate or a combination thereof. In another example, the fabric conditioner is a brightening agent that contains hydrogen peroxide, potassium permanganate, sodium peroxide, sodium perborate, disulfonated diaminostilbene optical brightener compounds and triazole optical brightener compounds. In yet another embodiment, the fabric conditioner is a bodying agent that is selected from among carboxymethyl cellulose, hydroxyethylcellulose, starch, polyvinyl acetate and combinations thereof. In yet another embodiment, the fabric conditioner is a wrinkle release agent that contains polyvinyl acetate.

Certain embodiments inlude fabric refresher spray compositions containing a composition for killing or repelling pests provided herein wherein the active ingredient is present at a concentration of from at or about 0.1% to at or about 5%, or in an amount of at or about 1% to at or about 10%, or greater than 10%, or greater than 15%, or greater than 20% or greater than 25% by weight of the composition. The fabric refresher spray compositions can further contain a cyclodextrin; and/or an ampholytic surfactant, an anionic surfactant, a cationic surfactant, a nonionic surfactant or a zwitterionic surfactant or a combination thereof; and/or water.

Certain embodiments include moist towelette products containing a substrate and a solution or emulsion of a composition comprising an insect or acarid repellant provided herein wherein the active ingredient is present in an amount of at least 0.1% by weight of the solution. The moist towelette products can further contain a surfactant containing, but not limited to, cocamidopropyl betaine, coco-glucoside or decyl glucoside or combinations thereof. In one embodiment, the substrate in the moist towelette product is a nonwoven fabric or cellulosic material.

Certain embodiments also include packaged pest repelling compositions that contain a container holding a composition comprising an insect or acarid repellant provided herein or an absorbent sheet impregnated with a composition comprising an insect or acarid repellant provided herein. Other embodiments include aerosol propellant pressurized sprayable pest repellents or pesticide products containing a composition comprising an insect or acarid repellant provided herein. The propellant in the aerosol propellant pressurized sprayable pest repellents or pesticide products can contain carbon dioxide, propane, butane or a mixture thereof.

Certain embodiments include methods of repelling an insect or pest from a location using a composition comprising an insect or acarid repellant provided herein. In the provided methods, a composition comprising an insect or acarid repellant provided herein is deployed at the location in an insect or pest repelling amount, wherein the insect or pest is repelled when the insect or pest comes into contact with the composition or vapors from the composition. In certain embodiments, the composition can be deployed by atomizing, brushing on, coating, dipping, drenching, dripping, dusting, foaming, infusing, injecting into or onto, pouring, rolling on, scattering, spraying, spreading, sprinkling or wiping the composition onto at least a portion of the location.

In certain embodiments of the method for repelling an insect or pest from a location, the location is the surface of the body of a human or animal. For example, the animal can be a companion animal or a farm animal. In other embodiments of the method, the composition is deployed by applying topically to an article of clothing of a human. In other embodiments of the method, the composition comprising an insect or acarid repellant provided herein is deployed by laundering an article of clothing of a human with a detergent or fabric softener or both that contains the composition. In yet another embodiment of the method, the composition is deployed by drying an article of clothing of a human with a fabric softener that contains the composition. The fabric softener can be provided as a dryer sheet or gel. In another embodiment of the method, the location is a surface that is skin, hair or fur and the composition is deployed by applying topically to the skin, hair or fur. The topically applied composition can be provided as an aerosol, a solution, an emulsion, an oil, a lotion, a soap, a spray, or a gel. In yet another embodiment of the method, the composition is provided to a surface that is skin, hair or fur in a form selected from among skin conditioners, hand/body/facial lotions, skin moisturizers, skin toners, skin sanitizers, skin cleansing compositions, skin soothing and lubricating compositions, sunscreen products, anti-aging products, tanning products, self-tanning products, after-sun products, masking products, anti-wrinkle products, hair conditioners, hair styling gels, hair anti-dandruff compositions, hair growth promoter compositions, hair lotions, hair tonics, rinses, conditioners, hair colorant compositions, hair anti-frizzing agent compositions, hair shining compositions, mousses, styling gels, hair pomade products and hair sprays, soaps, foaming bath products, hand/body/facial cleansers, astringent cleansers, anti-acne products, shampoos, body shampoos, synthetic detergent bars, shower gels and shampoos.

In certain embodiments of the method for repelling an insect or pest from a location, the location is a bedding location. The composition comprising an insect or acarid repellant provided herein can be deployed onto bedding, bed boards, bed slats, a mattress, box springs, furniture, carpeting, baseboards or flooring or a combination thereof by atomizing, coating, dipping, drenching, dripping, dusting, foaming, infusing, injecting into or onto, pouring, rolling on, scattering, spraying, spreading, sprinkling or wiping. In another embodiment of the method, the composition comprising an insect or acarid repellant provided herein is deployed by spraying the composition on to the surface of bedding, bed boards, bed slats, a mattress, box springs, furniture, carpeting, baseboards or flooring or a combination thereof. For example, the composition comprising an insect or acarid repellant provided herein can be deployed by injecting the composition into the mattress, box springs, furniture, carpeting, baseboards or flooring or a combination thereof. In certain embodiments of the method, the location contains a wood structure, wooden object or wall space.

In other embodiments of the method, the location is selected from among an air supply duct, an attic, an awning, a basement, a cellar, a crawlspace, a deck, a dock, a garage, a hamper, a heating vent, a home foundation, a linen storage closet, a pool deck, roof tiles, a shipping container, a storage unit, a suitcase, a walkway and a wall space and the composition is deployed by atomizing, coating, dipping, drenching, dripping, dusting, foaming, infusing, injecting into or onto, pouring, rolling on, scattering, spraying, spreading, sprinkling or wiping the composition onto or into at least a portion of the location. For example, the composition comprising an insect or acarid repellant provided herein is applied by spraying the composition onto a surface or is provided in the form of a powder and is applied by sprinkling the powder composition onto a surface. In yet other embodiments of the method for repelling an insect or pest from a location the composition comprising an insect or acarid repellant provided herein is deployed by providing an absorbent substrate or gel containing the composition and positioning it in the location. In one example, the absorbent substrate contains a nonwoven fabric or cellulosic material.

In certain embodiments, the insect or pest can be selected from among, but not limited to, ants, bedbugs, carpet beetles, centipedes, chiggers, drain flies, dust mites, earwigs, fleas, flies, gnats, hornets, lice, millipedes, mites, mosquitoes, roaches, scabies, silverfish, spiders, stinkbugs, termites, ticks, wasps, weevils and yellow jackets. For example, in the provided method, the insects or pests are ants that are selected from among Argentine ants, black ants, carpenter ants, fire ants, odorous house ants, pavement ants and pharaoh ants. In another embodiment, the insects or pests are lice that are selected from among head lice, body lice, pubic lice and nits thereof.

In certain embodiments, a composition comprising an insect or acarid repellant provided herein can be deployed by applying topically to an article of clothing of a human; or applying topically to skin or hair of a human; or applying topically to skin or fur of an animal. In some embodiments, the animal can be selected from among a bovine, canine, caprine, cervine, cricetine, feline, galline, equine, lapine, murine, musteline and ovine. For example, the animal is a companion animal. In other embodiments, the composition comprising an insect or acarid repellant provided herein can be deployed by laundering an article of clothing of a human with a detergent or fabric softener or both that contains the composition; or drying an article of clothing of a human with a fabric softener that contains the composition.

Certain embodiments include methods for repelling bedbugs, where a composition comprising an insect or acarid repellant provided herein is deployed by applying directly to the surface of the bedbugs or to bedding, bed boards, bed slats, a mattress, box springs, furniture, carpeting, baseboards or flooring or a combination thereof. For example, application of the composition comprising an insect or an acarid repellant provided herein can include spraying the composition on to the surface of bedding, bed boards, bed slats, a mattress, box springs, furniture or carpeting; or injecting the composition into the mattress, box springs, furniture or carpeting or a combination thereof; or deploying an absorbent substrate or gel containing the composition in the vicinity of bed boards, bed slats, a mattress, box springs, furniture or carpeting so that vapors from the composition come into contact with a surface of the bed boards, bed slats, a mattress, box springs, furniture or carpeting; or injecting the composition into a wall space. In certain embodiments, the absorbent substrate contains a nonwoven fabric or cellulosic material.

In one embodiment of the provided method for repelling bedbugs, the composition comprising an insect or acarid repellant provided herein contains from at or about 0.01% to at or about 10%, or greater than 10%, or greater than 15%, or greater than 20%, or greater than 25%, or up to 99% of an active ingredient, fatty acid, and a carrier. In some examples, the composition is formulated for delivery of an active ingredient for at least 4 days. In any of the methods provided herein, the composition can be formulated for delivery of an active ingredient for at least 1 day, or at least 2 days, or at least 3 days, or at least 4 days, or at least 5 days, or at least 6 days, at least 7 days, or at least 8 days, or at least 9 days, or at least 10 days, or at least 11 days, or at least 12 days, at least 13 days, for at least 14 days, or at least 15 days, or at least 16 days, or at least 17 days, or at least 18 days, or at least 19 days, at least 20 days, or at least 21 days, or at least 22 days, or at least 23 days, or at least 24 days, or at least 25 days, at least 26 days, or at least 27 days, or at least 28 days, or at least 29 days, or at least 30 days, or at least 31 days, or at least 45 days, or at least 60 days or at least 75 days or at least 90 days.

Certain embodiments include a method of preventing skin injury due to biting insects or pests by providing a composition for killing or repelling insects provided herein and applying the composition to a surface, wherein the insect or pest is repelled from the surface when it comes into contact with the composition comprising an insect or acarid repellant provided herein or with vapors from the composition comprising an insect or acarid repellant provided herein. In some embodiments the surface is clothing or bedding and the composition comprising an insect or acarid repellant provided herein can be applied to the surface by atomizing, coating, dipping, drenching, dripping, dusting, foaming, infusing, injecting into or onto, pouring, rolling on, scattering, spraying, spreading, sprinkling or wiping an amount of the composition onto the surface. In other embodiments of the provided method, the composition comprising an insect or acarid repellant provided herein is applied by washing the clothing or bedding with the composition comprising an insect or an acarid repellant provided herein that is provided as a detergent composition or a fabric softener composition or both. In other embodiments of the provided method, the composition comprising an insect or acarid repellant provided herein can be applied by drying the clothing or bedding with the composition that is provided as a fabric softener composition. In some embodiments of the method, the surface is bed boards, bed slats, a mattress, box springs, furniture or carpeting and the composition can be applied by atomizing, coating, dipping, drenching, dripping, dusting, foaming, infusing, injecting into or onto, pouring, rolling on, scattering, spraying, spreading, sprinkling or wiping the composition onto the surface. In yet another embodiment of the method, the composition comprising an insect or acarid repellant provided herein can be provided in the form of a powder and is applied by sprinkling the powder composition onto the surface.

In some embodiments of a method for preventing skin injury due to biting insects or pests, the surface is skin or hair of a human and the composition comprising an insect or acarid repellant provided herein can be applied topically to the skin or hair. In an embodiment, the composition comprising an insect or acarid repellant provided herein can be provided as an aerosol, a solution, an emulsion, an oil, a lotion, a soap, a spray, or a gel. In yet other embodiment, the composition is provided in a form selected from among skin conditioners, hand/body/facial lotions, skin moisturizers, skin toners, skin sanitizers, skin cleansing compositions, skin soothing and lubricating compositions, sunscreen products, anti-aging products, tanning products, self-tanning products, after-sun products, masking products, anti-wrinkle products, hair conditioners, hair styling gels, hair anti-dandruff compositions, hair growth promoter compositions, hair lotions, hair tonics, rinses, conditioners, hair colorant compositions, hair anti-frizzing agent compositions, hair shining compositions, mousses, styling gels, hair pomade products and hair sprays, soaps, foaming bath products, hand/body/facial cleansers, astringent cleansers, anti-acne products, shampoos, body shampoos, synthetic detergent bars, shower gels and shampoos. The insect or pest can be selected from ants, bedbugs, chiggers, fleas, lice, mites, mosquitoes, roaches, scabies, and ticks.

The disclosed invention can be a consumer care product prepared according to the process comprising (a) mixing an active ingredient and fatty acid; and (b) adding the mixture to a pharmaceutically suitable topical carrier.

In at least one embodiment, the disclosed invention includes a repellent pheromone being an anthranilate ester, alone or in combination with aromatic oils. Preferably, the repellent pheromone is present in the amounts of 4% to 10% w/w. In another embodiment, the fatty acids of the presently claimed consumer care product include saturated and unsaturated fatty acids containing from 8 to 24 carbon atoms, with fatty acids containing from 13 to 21 carbon atoms being preferred. Illustrative of the fatty acids which may be employed are oleic acid, ricinoleic acid, linoleic acid palmitic acid and stearic acid; with steric and oleic acids being particularly preferred.

In one embodiment, the present invention concerns a topical skin care composition that contains water, a surfactant, emulsifying agent, a skin conditioning agent or an emollient, a humectant, silicon, an anti-inflammatory, a sun blocking agent, a topical anesthetic, vitamins, an antipruritic, an antibiotic, antifungal, an antiseptic, a vasoconstrictor, and other suitable topical or cosmetic excipients such as a pH modifier, a thickener, an anti-oxidant, a preservative and the like.

In certain embodiments, the composition further contains a dispersing agent that is selected from among, but not limited to, a surfactant, polyvinyl pyrrolidone, polyoxyethylated castor oil, a polyoxyethylene sorbitan ester, alkylnaphthalene sulfonate, alkylbenzenesulfonate, polyoxyethylene, polycarboxylate, lignin sulfonate, sodium silicate, potassium silicate, methylcellulose, carboxymethyl cellulose, hydroxypropylcellulose, hydroxypropyl-methylcellulose, gum arabic, a polyacrylate, and an acrylic/maleic copolymers and combinations thereof.

In at least one embodiment, the composition contains a surfactant in amounts of 0 to 40% w/w, or an emulsifying agent including, but not limited to, such compounds as anionics, cationics, non-ionic, amphoteric, zwitteronics, and combinations thereof. Exemplary of such surfactants and/or emulsifiers suitable for use with the present disclosure include, but are not limited to, esters, amides, polysaccharide ethers, polyglycosides, fatty acids, fatty alcohols, amine oxides, water-soluble cellulose derivatives, alkyl sulfonates, ethoxylated alkyl phenols, alkanolamides, betaines, zwitterionic surfactants, carboxylated alcohols, carboxylic acids, ethoxylated alcohols, preferably ethoxylated C₁₂-C₁₅ alcohols (ex. Rhodasurf^{®} L-9 brand); nonionic surfactants such as polysorbate 20, polysorbate 80, alkoxylated alcohol, a dialkoxylated alcohol, an alkoxylated dialkylphenol, an alkylpolyglycoside, an alkoxylated alkylphenol, an alkoxylated glycol, an alkoxylated mercaptan, an alkylamine salt, an alkyl quaternary amine salt, a glyceryl or polyglyceryl ester of a natural fatty acid, an alkoxylated glycol ester, an alkoxylated fatty acid, an alkoxylated alkanolamide, a polyalkoxylated silicone and an N-alkyl pyrrolidone and combinations thereof, anionic surfactants such as DEA phosphate, alkyl sulfonate surfactants, a linear alkylbenzene sulfonic acid, a branched alkylbenzene sulfonic acid a C12 to C18 alkylsulfate, C12-C18 alkyl alkoxy sulfate, C12-C18 alkyl methyl ester sulfonate and combinations thereof, cationic surfactants such as behentrimonium methosulfate, alkylamine, an alkyl diamine, an alkyl polyamine, a mono- or di-quaternary ammonium salt, a monoalkoxylated amine, a dialkoxylated amine, a monoalkoxylated quaternary ammonium salt, a dialkoxylated quaternary ammonium salt, an etheramine, an amine oxide, an alkoxylated amine oxide and a fatty imidazoline and the like and derivatives thereof.

The present invention is in the form of a personal care composition, such as a foaming composition that contains single emulsifier or a combination of two or more emulsifiers. In at least one embodiment, the formulation contains both an aqueous and an organic phase, wherein the organic phase contains a surfactant and an organic diluent. In one embodiment, the organic phase may comprise the active ingredient, isopropyl myristate and ethoxylated castor oil or the like (ex. Agnique^{®}BP-4-3101 brand).

The dispersing agent can be present in the provided compositions in an amount of at or about 0.002% to at or about 50% by weight of the composition. In some examples, the dispersing agent is present in an amount of at or about 0.025% to at or about 25% by weight of the composition. In other examples, the dispersing agent is present in an amount of at or about 0.01% to at or about 15% by weight of the composition. In yet other examples, the dispersing agent is present in an amount of at or about 0.05% to at or about 10% by weight of the composition, for example, in an amount at or about at least 0.05%, 0.06%, 0.07%, 008%, 0.09%, 0.1%, 0.15%, 0.2%, 0.25%, 0.3%, 0.35%, 0.3%, 0.35%, 0.5%, 0.55%, 0.6%, 0.65%, 0.7%, 0.75%, 0.8%, 0.85%, 0.9%, 0.95%, 1%, 1.05%, 1.1%, 1.15%, 1.2%, 1.25%, 1.3%, 1.35%, 1.4%, 1.45%, 1.5%, 1.55%, 1.6%, 1.65%, 1.7%, 1.75%, 1.8%, 1.85%, 1.9%, 1.95%, 2%, 2.05%, 2.1%, 2.15%, 2.2%, 2.25%, 2.3%, 2.35%, 2.4%, 2.45%, 2.5%, 2.55%, 2.6%, 2.65%, 2.7%, 2.75%, 2.8%, 2.85%, 2.9%, 2.95%, 3%, 3.05%, 3.1%, 3.15%, 3.2%, 3.25%, 3.3%, 3.35%, 3.4%, 3.45%, 3.5%, 3.55%, 3.6%, 3.65%, 3.7%, 3.75%, 3.8%, 3.85%, 3.9%, 3.95%, 4%, 4.05%, 4.1%, 4.15%, 4.2%, 4.25%, 4.3%, 4.35%, 4.4%, 4.45%, 4.5%, 4.55%, 4.6%, 4.65%, 4.7%, 4.75%, 4.8%, 4.85%, 4.9%, 4.95%, 5%, 5.05%, 5.1%, 5.15%, 5.2%, 5.25%, 5.3%, 5.35%, 5.4%, 5.45%, 5.5%, 5.55%, 5.6%, 5.65%, 5.7%, 5.75%, 5.8%, 5.85%, 5.9%, 5.95%, 6%, 6.05%, 6.1%, 6.15%, 6.2%, 6.25%, 6.3%, 6.35%, 6.4%, 6.45%, 6.5%, 6.55%, 6.6%, 6.65%, 6.7%, 6.75%, 6.8%, 6.85%, 6.9%, 6.95%, 7%, 7.05%, 7.1%, 7.15%, 7.2%, 7.25%, 7.3%, 7.35%, 7.4%, 7.45%, 7.5%, 7.55%, 7.6%, 7.65%, 7.7%, 7.75%, 7.8%, 7.85%, 7.9%, 7.95%, 8%, 8.05%, 8.1%, 8.15%, 8.2%, 8.25%, 8.3%, 8.35%, 8.4%, 8.45%, 8.5%, 8.55%, 8.6%, 8.65%, 8.7%, 8.75%, 8.8%, 8.85%, 8.9%, 8.95%, 9%, 9.05%, 9.1%, 9.15%, 9.2%, 9.25%, 9.3%, 9.35%, 9.4%, 9.45%, 9.5%, 9.55%, 9.6%, 9.65%, 9.7%, 9.75%, 9.8%, 9.85%, 9.9%, 9.95% and 10% by weight of the composition.

In another embodiment, the composition may contain one or more thickening agents. In one embodiment, a thickening agent is present at a level of from about 0 to about 40 %, preferably from about 0.1% to about 30 %, and more preferably from about 0.25% to about 15%, by weight of the composition. Non-limiting classes of thickening agents include those selected from the following: carboxylic acid polymers useful such as carbomers, crosslinked acrylic acid with allyl ethers of sucrose or pentaerythritol, generally available as the Carbopol series from B.F. Goodrich. Other thickening agent include crosslinked polyacrylate polymers, polyacrylamide polymers, polysaccharides or gelling agents such as cellulose, carboxymethyl hydroxyethylcellulose, cellulose acetate propionate carboxylate, hydroxyethylcellulose, hydroxyethyl ethylcellulose, hydroxypropylcellulose, hydroxypropyl methylcellulose, methyl hydroxyethylcellulose, microcrystalline cellulose, sodium cellulose sulfate, and mixtures thereof. Other thickening agents include gums such as acacia, agar, algin, alginic acid, ammonium alginate, amylopectin, calcium alginate, calcium carrageenan, carnitine, carrageenan, dextrin, gelatin, gellan gum, guar gum, guar hydroxypropyltrimonium chloride, hectorite, hyaluronic acid, hydrated silica, hydroxypropyl chitosan, hydroxypropyl guar, karaya gum, kelp, locust bean gum, natto gum, potassium alginate, potassium carrageenan, propylene glycol alginate, sclerotium gum, sodium carboxymethyl dextran, sodium carrageenan, tragacanth gum, xanthan gum (Kelzan^{®}), and mixtures thereof.

In at least one embodiment the topical composition may further contain one or more topical anesthetics (or a plurality of topical anesthetics). Preferably, the topical anesthetic is lidocaine. In other embodiments, in lieu of, or in addition to, lidocaine, one or more of the following may be used: benzocaine, butamben, dibucaine, oxybuprocaine, pramoxine, proparacaine, proxymetacaine, tetracaine, amethocaine, propoxycaine, mepivacaine, bupivacaine, and the like, and mixtures thereof and equivalents.

In at least another embodiment, the anti-inflammatory agent can be a nonsteroidal such as diclofenac, salicylate naproxen, ketorolac and the like or a corticosteroid a such as hydrocortisone, prednisone, cortisol; dexamethasone, alcometasone; hydrocortisone 21-acetate; hydrocortisone 17-valerate; hydrocortisone 17-butyrate; bethamethasone 17,21-dipropionate; descinolone acetonide; bethamethasone valerate; prednisolone; fluorometholone; flucloronide; cortisone acetate; flunisolide acetate; triamcinolone acetonide; flucinonide; desconide; and the like; and mixtures thereof; and equivalents thereof. It is to be understood that any anti-inflammatory agent and/or topical corticosteroid may be used in the composition of the present invention. In other embodiments, the anti-inflammatory agent/topical corticosteroid is omitted.

In yet another embodiment, the composition comprises one or more anti-fungal agents. Preferably, the anti-fungal agent is nystatin; lotrimazole; fluconazole; ketoconazole; itraconazole; lotrimin; canesten; mycosporin; mycelex; clotrimazol; mykosporin; empecid; chlotrimazole, and the like, and mixtures thereof and equivalents thereof.

In yet another embodiment, the composition may further comprise a sun block or UV light blocker such as para-aminobenzoic acid (PABA), PABA esters (glyceryl PABA, amyldimethyl PABA and octyldimethyl PABA), butyl PABA, ethyl PABA, ethyl dihydroxypropyl PABA, benzophenones (oxybenzone, sulisobenzone, benzophenone, and benzophenone-1 through 12), avobenzone, cinnamates (and octyl methoxycinnamate, isoamyl p-methoxycinnamate, octylmethoxy cinnamate, cinoxate, diisopropyl methyl cinnamate, DEA-methoxycinnamate, ethyl diisopropylcinnamate, glyceryl octanoate dimethoxycinnamate and ethyl methoxycinnamate), cinnamate esters, salicylates (homomethyl salicylate, benzyl salicylate, glycol salicylate, isopropylbenzyl salicylate, etc.), trolamine salicylate, anthranilates, ethyl urocanate, homosalate, dibenzoylmethane derivatives (e.g., avobenzone), octocrylene, methylbenzylidene camphor, etc. and the brands Tinosorb M^{™}, Tinosorb S^{™}, Tinosorb A2B^{™}, Neo Helipan^{™}, Mexoryl XL^{™}, Uvinul T^{™}, Parsol SLX^{™}. Non-limiting examples of physical sunblocks include, kaolin, talc, petrolatum and metal oxides (e.g., titanium dioxide and zinc oxide or the like. Additional examples of skin conditioning agents, UV light blockers, thickeners, antioxidants, and pharmaceutically and cosmetically acceptable ingredients may be found in such texts as McCutcheons Emulsifiers and Detergents as well as (Vol 2 ) Functional Materials, both the North American Editions and the International Editions, published yearly by McCutcheon Publications.

In yet another embodiment, the topical composition can further contain a skin conditioning agent or an emollient including a plant and/or a herbal component to make the external layers of the skin softer and more pliable. The emollient may be aloe vera extract, rose oil (i.e., the essential oils extracted from various types of rose), arnica extract, chamomile extract; chamomile flower extract; cetearyl alcohol; isopropyl myristate; triglyceride; myristic acid; palmitic acid; PEG-60 hydrogenated castor oil; glceryl linoleate; cyclomethicone; dimethicone; decyl oleate; stearic acid; lanolin; plant oils; shea butter; cocoa butter; algae extract; avocado oil; seed oil; buckthorn; calendula; camellia oil; capsaicin; castor oil; chamomile oil; lemongrass extract; lime oil; marula oil; olive oil; panthenol; corn oil; flaxseed oil; ginseng extract; haxel oil; pomegranate seed oil; rice bran oil; ginger; and the like, and mixtures thereof. In another embodiment, the topical composition may contain a vitamin such as vitamin A, B, C, E, beta-carotene; vitamin B complexes; vitamin B12, an antioxidant, a mineral such as sodium, potassium, calcium or the like. In other embodiments, the vitamin or minerals may be omitted.

Non-limiting examples of antioxidants that can be used with the compositions of the present invention include acetyl cysteine, ascorbic acid polypeptide, ascorbyl dipalmitate, ascorbyl methylsilanol pectinate, ascorbyl palmitate, ascorbyl stearate, BHA, BHT, t-butyl hydroquinone, cysteine, cysteine HCI, diamylhydroquinone, di-t-butylhydroquinone, dicetyl thiodipropionate, dioleyl tocopheryl methylsilanol, disodium ascorbyl sulfate, distearyl thiodipropionate, ditridecyl thiodipropionate, dodecyl gallate, erythorbic acid, esters of ascorbic acid, ethyl ferulate, ferulic acid, gallic acid esters, hydroquinone, isooctyl thioglycolate, kojic acid, magnesium ascorbate, magnesium ascorbyl phosphate, methylsilanol ascorbate, natural botanical anti-oxidants such as green tea or grape seed extracts, nordihydroguaiaretic acid, octyl gallate, phenylthioglycolic acid, potassium ascorbyl tocopheryl phosphate, potassium sulfite, propyl gallate, quinones, rosmarinic acid, sodium ascorbate, sodium bisulfite, sodium erythorbate, sodium metabisulfite, sodium sulfite, superoxide dismutase, sodium thioglycolate, sorbityl furfural, thiodiglycol, thiodiglycolamide, thiodiglycolic acid, thioglycolic acid, thiolactic acid, thiosalicylic acid, tocophereth-5, tocophereth-10, tocophereth-12, tocophereth-18, tocophereth-50, tocopherol, tocophersolan, tocopheryl acetate, tocopheryl linoleate, tocopheryl nicotinate, tocopheryl succinate, and tris(nonylphenyl) phosphite

Examples of preservatives include quaternary ammonium preservatives such as polyquaternium-1 and benzalkonium halides (e.g., benzalkonium chloride ("BAC") and benzalkonium bromide), parabens (e.g., methylparabens and propylparabens), phenoxyethanol, benzyl alcohol, chlorobutanol, phenol, sorbic acid, thimerosal, an azole, benzisothiazolin-3-one, benzalkonium quaternary compounds, benzyl alcohol, borates, 2-bromo-2-nitro-propane-1,3-diol, butylparaben, 5-chloro-2-methyl-4-isothiazolin-3-one, chlorphenesin, chlor-oxylenol, diazolidinyl urea, a dimethyl-benzylalkyl-ammonium chloride, ethyl paraben, formaldehyde, glutaraldehyde, halogenated salicylanilides, hexachlorophene, isobutyl-paraben, isothiazolin-3-one, 2-methyl-4-isothiazoline-3-one, methylparaben, mono-chloracetamide, neomycin sulfate, o-phenylphenol and salts thereof, phenoxyethanol, propionic acid and salts thereof, propylparaben, sodium benzoate, sorbic acid and salts thereof, tebuconazole and triazoles, or combinations thereof.

In certain embodiments, the compositions provided herein can further contain a colorant that is selected from among, but not limited to, a dye or pigment. In one example, the colorant is present in an amount at or about 0.0001% to at or about 1% by weight of the composition. In another example, the colorant is present in an amount at or about 0.0005% to at or about 0.5% by weight of the composition.

The compositions for killing or repelling pests provided herein can be formulated as a personal care or cosmetic composition. In some applications, the personal care or cosmetic composition is formulated as a product selected from among insect repellents, skin care products, hair care products, and cleansing products. In some examples, the personal care or cosmetic composition is a skin care product that is selected from among skin conditioners, hand/body/facial lotions, skin moisturizers, skin toners, skin sanitizers, skin cleansing compositions, skin soothing and lubricating compositions, sunscreen products, anti-aging products, tanning products, self-tanning products, after-sun products, masking products and anti-wrinkle products. In other examples, the personal care or cosmetic composition is a hair care product that is selected from among hair conditioners, hair styling gels, hair anti-dandruff compositions, hair growth promoter compositions, hair lotions, hair tonics, rinses, conditioners, hair colorant compositions, hair anti-frizzing agent compositions, hair shining compositions, mousses, styling gels, hair pomade products and hair sprays. In other examples, the personal care or cosmetic composition is a cleansing product that is selected from among soaps, foaming bath products, hand/body/facial cleansers, astringent cleansers, anti-acne products, shampoos, body shampoos, synthetic detergent bars, shower gels and shampoos.

In at least one embodiment, the topical composition can contain a repellent component from 0.01 % to about 99% by weight. In at least one embodiment, the repellent component can range from 0.01 to 25 wt %. In one preferred embodiment, the repellant is methyl anthranilate (also may be referred herein as MA ) in ranges of about 2 to 15 wt %. In a more preferred embodiment, the amount of methyl anthranilate is below 8% and can be applied to a subject's skin in an amount up to about 5000 mg/kg.

In at least one embodiment, the topical formulation contains an organic and an aqueous phase. In one embodiment, the organic phase contains the repellant and optionally a surfactant or an emulsifying agent in combination with a fatty acid. In a preferred embodiment, the aqueous phase contains water, glycerin, lecithin, a C₁-C₆ alcohol or any combinations thereof. In an alternative embodiment, the aqueous phase may contain a thickening agent such as xanthan gum. In one embodiment, the aqueous phase may contain water in an amount ranging from about 5 % to about 50 %, preferably in the ranges of 40% to 45% and xanthan gum in an amount of 0 % to about 40%, preferably in the range of 20% to 30%, all % in % w/w.

In at least one embodiment, the topical formulation is in the form of an oil in water emulsion containing MA, oleic acid, steric acid, ethoxylated alcohol (e.x. Rhodasur^{®} L-9 brand), glycerin and water. In at least one preferred embodiment, the MA is in the ranges of about 2% to about 8 %, preferably 3.35% to 6.7 %. In another embodiment, oleic acid is in the ranges of about 0.5 to 3 %, preferably 0.065% to 1.3 %. In another embodiment, steric acid can be in the ranges of about 1 to 10 wt%, preferably in the ranges of 1.5 to 4.0 t%, all % are % w/w.

In at least another embodiment, the topical formulation contains diethylhexyl adipate, homosalate, octocrylene, octisalate, avobenzone, bemotrizinol, cyclopentasiloxane, cyclohexasiloxane, necromone and ethanol. In yet another embodiment, the formulation is in the form of a lotion containing water and lecithin in the aqueous phase, while containing oleic acid, methyl anthranilate and Caproyl 90^{™} in the organic phase. In yet another embodiment, the topical formulation is in the form of a body spray containing C₁₂-C₁₅ alkyl benzoate, triethylhexyl citrate, isododecane, necromone, polyamide-3 and denatured alcohol.

In at least one embodiment, the consumer care product comprises MA in an amount ranging from about 1% to about 10%; about 3% to about 7.5%; 3.35% to about 6.7%. In another embodiment, oleic acid (also refered herein as "OA") is in an amount ranging from about 0.1% to about 2.5%, preferably about 0.45% to about 1.3%; steric acid in an amount ranging from about 1% to about 8%, preferably about 2.0% to about 4.0 %; an ethoxylated C₁₂-C₁₅ alcohol in an amount ranging from about 1.5% to about 12%; preferably about 3.0% to about 6%, glycerin in amount of about 2.5 to about 15%; preferably about 5% and a solvent such as water in an amount of 25% to 85%, preferably about 75%, all % are % w/w.

Another aspect of the present invention is directed to a method of repelling insect and/or acarid pests employing the anthranilate ester/fatty acid compositions described above. Such compositions provide effective repellent activity against a number of insect orders including Hymenoptera, including pavement ant, carpenter ant, Argentine ant, little fire ant, ghost ant, cold tolerant ant, wood ant, Asian needle ant, odorus ant, rover ant, fire ant, bigheaded ant, honeybee, carpenter bee, bumble bee, small carpenter bee, European paper wasp, German yellowjacket and bald-faced hornet; Blattodea including German roach, Eastern subterranean termite; Hemiptera including bed bugs and brown marmorated stink bugs; Diptera including Aedes and Anopheles mosquitoes; Coleoptera including corn rootworm, sawtoothed beetle and confused flour beetle; Siphonaptera including cat flea and Lepidoptra including tobacco budworm. Repellent activity has also been demonstrated against brown dog ticks.

The compositions of the present invention have been shown not to exhibit repellent activity against a number of bird species including hummingbirds, grackles, sparrows, woodpeckers, jays, chickadees, titmouse, wrens, starlings, cardinals, finches, flickers and doves.

In at least another embodiment, the present invention is directed to a non-therapeutic method of repelling an insect from a subject comprising topically administering to a subject in need thereof a composition comprising at least one insect or acarid repellant component containing an anthranilate ester and a pharmaceutically suitable topical carrier. In one embodiment, the subject is a mammal such as human, dogs, other domesticated animals or live cattle.

In another embodiment, the present topical compositions can be directly applied to the skin or a user's apparel and clothing. In yet another embodiment, the insect orders including Hymenoptera, including pavement ant, carpenter ant, Argentine ant, little fire ant, ghost ant, cold tolerant ant, wood ant, Asian needle ant, odorus ant, rover ant, fire ant, bigheaded ant, honeybee, carpenter bee, bumble bee, small carpenter bee, European paper wasp, German yellowjacket and bald-faced hornet; Blattodea including German roach, Eastern subterranean termite; Hemiptera including bed bugs and brown marmorated stink bugs; Diptera including Aedes and Anopheles mosquitoes; Coleoptera including corn rootworm, sawtoothed beetle and confused flour beetle; Siphonaptera including cat flea and Lepidoptra including tobacco budworm and brown dog ticks.

Preferred embodiments of the invention include: on-skin formulations containing a mixture of oleaic acid and methyl anthranilate, in the various proportions recited herein; household products such as sprays containing a mixture of oleaic acid and methyl anthranilate, in the various proportions recited herein; and household products such as clothing treatments containing a mixture of oleaic acid and methyl anthranilate, in the various proportions recited herein.

The modifier "about" is used herein to indicate that certain preferred operating ranges, such as ranges for molar ratios for reactants, material amounts, and temperature, are not fixedly determined. The meaning will often be apparent to one of ordinary skill. For example, a recitation of a concentration of about 200 grams per liter in reference to, for example, a formulation would be interpreted to include other like concentrations that can be expected to provide similar effect for the concentration, such as 180 grams per liter or 220 grams per liter. Where guidance from the experience of those of ordinary skill is lacking, guidance from the context is lacking, and where a more specific rule is not recited below, the "about" range shall be not more than 10% of the absolute value of an end point or 10% of the range recited, whichever is less.

## Claims

1. A consumer care product comprising an insect- or acarid-repellant composition comprising:
(a) a first active ingredient selected from the group consisting of dimethyl anthranilate, menthyl anthranilate, ethyl anthranilate, phenylethyl anthranilate and methyl anthranilate;
(b) a fatty acid selected from the group consisting of oleic acid, ricinoleic acid, linoleic acid, palmitic acid, stearic acid and any mixtures thereof;
(c) optionally at least one additional active ingredient;
(d) a pharmaceutically suitable topical carrier; and optionally comprising
(e) an additional compound selected from the group consisting of a surfactant, emulsifying agent, a pH modifier, a thickening agent, an anti-oxidant, a preservative, a skin conditioning agent, an emollient, a humectant, a silicone moiety, an anti-inflammatory, a sun blocking agent, a topical anesthetic, vitamins, an antipruritic, an antibiotic, antifungal, an antiseptic, a vasoconstrictor, and any mixtures thereof;
wherein the weight ratio of first active ingredient to the fatty acid of part (b) is 3:1 or 6:1.

2. The consumer care product of claim 1, wherein the fatty acid is oleic acid

3. The consumer care product of claim 1 wherein the anthranilate ester is methyl anthranilate.

4. The consumer care product of claim 1, wherein the fatty acid is oleic acid and the anthranilate ester is methyl anthranilate.

5. The consumer care product of claim 1, wherein the emulsifying agent of part (e) is selected from the group consisting of polysaccharide ethers, polyglycosides, fatty acids, fatty alcohols, amine oxides, water-soluble cellulose, alkyl sulfonates, ethoxylated alkyl phenols, alkanolamides, betaines, zwitterionic surfactants, carboxylated alcohols, carboxylic acids, ethoxylated C₁₂-C₁₅ alcohols, polysorbates, behentrimonium methosulfate, isopropyl myristate, ethoxylated castor oil and any mixtures thereof.

6. The consumer care product of claim 1, wherein the thickening agent is selected from the group consisting of carboxylic acid polymers, crosslinked acrylic acid with allyl ethers of sucrose, crosslinked polyacrylate polymers, polyacrylamide polymers, polysaccharides, gums and any mixtures thereof.

7. The consumer care product of claim 7, wherein the polysaccharides are selected from the group consisting of cellulose, carboxymethyl hydroxyethylcellulose, cellulose acetate propionate carboxylate, hydroxyethylcellulose, hydroxyethyl ethylcellulose, hydroxypropylcellulose, hydroxypropylmethylcellulose, methyl hydroxyethylcellulose, microcrystalline cellulose, sodium cellulose sulfate, chitosan and any mixtures thereof.

8. The consumer care product of claim 8, wherein the pharmaceutically suitable topical carrier of part (d) comprises water, glycerin, lecithin, a fatty acid or any combinations thereof.

9. A non-therapeutic method for repelling insects or acarids by applying the composition of claim 1 to the surface of the body of a human subject or a livestock animal.

## Patentansprüche

1. Verbraucherpflegeprodukt, das eine insekten- oder akaridabwehrende Zusammensetzung umfasst, umfassend:
(a) einen ersten Wirkstoff, ausgewählt aus der aus Dimethylanthranilat, Menthylanthranilat, Ethylanthranilat, Phenylethylanthranilat und Methylanthranilat bestehenden Gruppe,
(b) eine Fettsäure, ausgewählt aus der aus Ölsäure, Ricinolsäure, Linolsäure, Palmitinsäure, Stearinsäure und beliebigen Mischungen davon bestehenden Gruppe,
(c) gegebenenfalls mindestens einen zusätzlichen Wirkstoff,
(d) einen pharmazeutisch geeigneten topischen Träger, und gegebenenfalls umfassend
(e) eine zusätzliche Verbindung, ausgewählt aus der aus einem Tensid, einem Emulgator, einem pH-Modifikator, einem Verdickungsmittel, einem Antioxidans, einem Konservierungsmittel, einem Hautpflegemittel, einem Weichmacher, einem Feuchthaltemittel, einem Silikonanteil, einem entzündungshemmenden Mittel, einem Sonnenschutzmittel, einem topischen Anästhetikum, Vitaminen, einem Mittel gegen Juckreiz, einem Antibiotikum, einem Antimykotikum, einem Antiseptikum, einem Vasokonstriktor und beliebigen Mischungen davon bestehenden Gruppe,
wobei das Gewichtsverhältnis von erstem Wirkstoff zu Fettsäure des Teils (b) 3:1 oder 6:1 beträgt.

2. Verbraucherpflegeprodukt nach Anspruch 1, wobei die Fettsäure Ölsäure ist.

3. Verbraucherpflegeprodukt nach Anspruch 1, wobei der Anthranilatester Methylanthranilat ist.

4. Verbraucherpflegeprodukt nach Anspruch 1, wobei die Fettsäure Ölsäure ist und der Anthranilatester Methylanthranilat ist.

5. Verbraucherpflegeprodukt nach Anspruch 1, wobei der Emulgator des Teils (e) aus der aus Polysaccharideethern, Polyglykosiden, Fettsäuren, Fettalkoholen, Aminoxiden, wasserlöslicher Cellulose, Alkylsulfonaten, ethoxylierten Alkylphenolen, Alkanolamiden, Betainen, zwitterionischen Tensiden, carboxylierten Alkoholen, Carbonsäuren, ethoxylierten C₁₂-C₁₅-Alkoholen, Polysorbaten, Behentrimoniummethosulfat, Isopropylmyristat, ethoxyliertem Rizinusöl und beliebigen Mischungen davon bestehenden Gruppe ausgewählt ist.

6. Verbraucherpflegeprodukt nach Anspruch 1, wobei das Verdickungsmittel aus der aus Carbonsäurepolymeren, vernetzter Acrylsäure mit Allylethern von Saccharose, vernetzten Polyacrylatpolymeren, Polyacrylamidpolymeren, Polysacchariden, Gummen und beliebigen Mischungen davon bestehenden Gruppe ausgewählt ist.

7. Verbraucherpflegeprodukt nach Anspruch 7, wobei die Polysaccharide aus der aus Cellulose, Carboxymethylhydroxyethylcellulose, Celluloseacetatpropionatcarboxylat, Hydroxyethylcellulose, Hydroxyethylethylcellulose, Hydroxypropylcellulose, Hydroxypropylmethylcellulose, Methylhydroxyethylcellulose, mikrokristalliner Cellulose, Natriumcellulosesulfat, Chitosan und beliebigen Mischungen davon bestehenden Gruppe ausgewählt sind.

8. Verbraucherpflegeprodukt nach Anspruch 8, wobei der pharmazeutisch geeignete topische Träger des Teils (d) Wasser, Glycerin, Lecithin, eine Fettsäure oder beliebige Kombinationen davon umfasst.

9. Nichttherapeutisches Verfahren zur Abwehr von Insekten oder Akariden durch Aufbringen der Zusammensetzung nach Anspruch 1 auf die Körperoberfläche eines menschlichen Subjekts oder eines Nutztieres.

## Revendications

1. Produit de soins de consommation comprenant une composition répulsive contre les insectes ou les acariens comprenant :
a) un premier ingrédient actif choisi dans le groupe constitué par l'anthranilate de diméthyle, l'anthranilate de menthyle, l'anthranilate d'éthyle, l'anthranilate de phényléthyle et l'anthranilate de méthyle ;
b) un acide gras choisi dans le groupe constitué par l'acide oléique, l'acide ricinoléique, l'acide linoléique, l'acide palmitique, l'acide stéarique et de quelconques mélanges de ceux-ci ;
c) éventuellement au moins un ingrédient actif supplémentaire ;
(d) un support topique pharmaceutiquement approprié ; et comprenant éventuellement
(e) un composé supplémentaire choisi dans le groupe constitué par un tensioactif, un agent émulsifiant, un modificateur de pH, un agent épaississant, un antioxydant, un conservateur, un agent de conditionnement de la peau, un émollient, un humectant, une fraction de silicone, un anti-inflammatoire, un agent de blocage solaire, un anesthésique topique, des vitamines, un antiprurigineux, un antibiotique, un antifongique, un antiseptique, un vasoconstricteur, et de quelconques mélanges de ceux-ci ;
dans lequel le rapport pondéral du premier ingrédient actif sur l'acide gras de la partie (b) est de 3:1 ou 6:1.

2. Produit de soins de consommation selon la revendication 1, dans lequel l'acide gras est l'acide oléique.

3. Produit de soins de consommation selon la revendication 1, dans lequel l'ester d'anthranilate est l'anthranilate de méthyle.

4. Produit de soins de consommation selon la revendication 1, dans lequel l'acide gras est l'acide oléique et l'ester d'anthranilate est l'anthranilate de méthyle.

5. Produit de soins de consommation selon la revendication 1, dans lequel l'agent émulsifiant de la partie (e) est choisi dans le groupe constitué par éthers de polysaccharides, polyglycosides, acides gras, alcools gras, oxydes d'amines, cellulose hydrosoluble, sulfonates d'alkyle, alkylphénols éthoxylés, alcanolamides, bétaïnes, tensioactifs zwitterioniques, alcools carboxylés, acides carboxyliques, alcools en C₁₂-C₁₅ éthoxylés, polysorbates, méthosulfate de béhentrimonium, myristate d'isopropyle, huile de ricin éthoxylée et de quelconques mélanges de ceux-ci.

6. Produit de soins de consommation selon la revendication 1, dans lequel l'agent épaississant est choisi dans le groupe constitué par les polymères d'acide carboxylique, l'acide acrylique réticulé avec des éthers allyliques de saccharose, les polymères de polyacrylate réticulés, les polymères de polyacrylamide, les polysaccharides, les gommes et de quelconques mélanges de ceux-ci.

7. Produit de soins de consommation selon la revendication 7, dans lequel les polysaccharides sont choisis dans le groupe constitué par la cellulose, la carboxyméthylhydroxyéthylcellulose, l'acétate propionate carboxylate de cellulose, l'hydroxyéthylcellulose, l'hydroxyéthyléthylcellulose, l'hydroxypropylcellulose, l'hydroxypropylméthylcellulose, la méthylhydroxyéthylcellulose, la cellulose microcristalline, le sulfate de cellulose sodique, le chitosane et de quelconques mélanges de ceux-ci.

8. Produit de soins de consommation selon la revendication 8, dans lequel le support topique pharmaceutiquement approprié de la partie (d) comprend l'eau, la glycérine, la lécithine, un acide gras ou toutes combinaisons de ceux-ci.

9. Procédé non thérapeutique pour repousser les insectes ou les acariens par application de la composition selon la revendication 1 sur la surface du corps d'un sujet humain ou d'un animal de bétail.
